(19)

Europäisches
Patentamt
European
Patent Office
Office européen
des brevets

(11)  EP 2 913 660 A1

(12)  EUROPEAN PATENT APPLICATION
published in accordance with Art. 153(4) EPC

(43) Date of publication:
02.09.2015 Bulletin 2015/36

(51) Int Cl.:
G01N 21/64 (2006.01)    G01N 21/76 (2006.01)
G01N 33/558 (2006.01)

(21) Application number: 13849687.2

(22) Date of filing: 04.07.2013

(86) International application number:
PCT/JP2013/068406

(87) International publication number:
WO 2014/064971 (01.05.2014 Gazette 2014/18)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME

(30) Priority: 25.10.2012 JP 2012235737

(71) Applicant: Olympus Corporation
Shibuya-ku
Tokyo 151-0072 (JP)

(72) Inventor: HANASHI, Takuya
Tokyo 151-0072 (JP)

(74) Representative: Gunzelmann, Rainer
Wuesthoff & Wuesthoff
Patentanwälte PartG mbB
Schweigerstraße 2
81541 München (DE)

(54)  **METHOD FOR DETECTING TARGET PARTICLES**

(57)  The invention provides a method of detecting a non-light emitting target particle in a solution using a scanning molecule counting method without applying a light emitting label. According to the invention, there is provided a target particle detection method of detecting a non-light emitting particle using an optical system of a confocal microscope or a multi-photon microscope, including the steps of: binding a non-light emitting observation particle and a solid phase carrier to each other through a target particle which is a detection target in a solution or at an interface of the solution; (b) removing observation particles in a free state after the step (a); (c) separating the observation particles from the complexes after the step (b); (d) preparing a sample solution for measurement in which the separated observation particles are dispersed after the step (c); and (e) detecting the observation particles in the sample solution for measurement prepared in the step (d) using an inverted scanning molecule counting method.

FIG. 2A

EP 2 913 660 A1

**Description**

Technical Field

The present invention relates to a method of detecting target particles using an optical system such as an optical system of a confocal microscope or a multi-photon microscope capable of detecting light from a small region in a solution.

Background Art

By virtue of recent development in optical measurement techniques, it is possible to detect and measure weak light of a single photon level or of a fluorescent single molecule level using an optical system of a confocal microscope and an ultrasensitive light detection technique capable of performing photon counting (one-photon detection). Thus, various devices and methods for detecting an intermolecular interaction or a binding/dissociation reaction of biomolecules and the like using such a weak light measurement technique have been proposed. Particularly, according to a method using a fluorescence measurement technique in a small region (referred to as a focal region where laser light of a microscope is condensed, that is, a confocal volume) that uses an optical system of a confocal microscope and a photon counting technique such as fluorescence correlation spectroscopy (FCS) and fluorescence-intensity distribution analysis (FIDA); a sample necessary for the measurement may be low in concentration and be small in amount compared to the related art. The amount of the sample which is used in a single measurement is approximately several tens of μL. Since a second-order measurement is repeated several times in a single measurement, the measurement time is also significantly reduced. Furthermore, as an aspect of the FCS, there is a method (inverted FCS (iFCS)) of calculating, in a system in which the light intensity detected when non-light emitting particles dispersed in a solution containing a large amount of light emitting substances dissolved therein enter a confocal volume is reduced, a value of an autocorrelation function of the fluorescence intensity, and calculating a translational diffusion time of the non-light emitting particles in the confocal volume and an average number of staying particles (for example, see Patent Document 1).

Recently, a new optical analysis technique (scanning molecule counting method) for individually detecting, using an optical system such as an optical system of a confocal microscope or a multi-photon microscope capable of detecting light from a small region in a solution, while moving the position of a small region which is a light detection region in a sample solution for measurement, light emitting particles (particles which emit light due to fluorescence, phosphorescence, chemiluminescence, bioluminescence, light scattering or the like) crossing the small region has been proposed (for example, see Patent Documents 2 to 4). Specifically, in the scanning molecule counting method, using the optical system of the confocal microscope or the multi-photon microscope, while moving the position of the light detection region of the optical system in the sample solution for measurement, the light emitted from the light emitting particles in the light detection region is detected. Accordingly, the light emitting particles in the sample solution for measurement are individually detected, and thus it is possible to acquire information related to counting of the light emitting particles and the concentration or the number density of the light emitting particles in the sample solution for measurement.

As in the optical analysis technique such as a FIDA, since the scanning molecule counting method has a light detection mechanism configured to detect the light from a light detection region of a confocal microscope or a multi-photon microscope, the amount of a sample solution for measurement may be small (for example, approximately several tens of μL), and the measurement time is short as in FIDA and the like. Differently from the FIDA or the like requiring a statistical process such as calculation of a fluctuation in the fluorescence intensity, in the scanning molecule counting method, such a statistical process is not executed. Therefore, the optical analysis technique of the scanning molecule counting method can be applied to a sample solution for measurement of which the number density or the concentration of particles is significantly lower than a level necessary for the optical analysis technique such as the FIDA. That is, by detecting target particles (observation target particles) labeled by a light-emitting probe in the sample solution for measurement using the scanning molecule counting method, the state or the characteristics of the target particles can be detected and analyzed even when the concentration or the number density of the target particles in the sample solution for measurement is extremely low (for example, see Patent Document 5).

Citation List

Patent Documents

Patent Document 1: International Publication No. 2010/119098

Patent Document 2: International Publication No. 2011/108369
Patent Document 3: International Publication No. 2011/108370
Patent Document 4: International Publication No. 2011/108371
Patent Document 5: International Publication No. 2012/014778

Summary of the Invention

Problems to be solved by the Invention

In a scanning molecule counting method of individually detecting light of single light emitting particles, since the light from the single particle is weak, it is easily influenced by stray light and Raman scattering of water. That is, in an analysis method of recognizing an increase in the light intensity value indicating the light emitted from the light emitting particle as a signal of the light emitting particle, the stray light and the light generated due to the Raman scattering of water may be erroneously recognized as a signal of the light emitting particle. In addition, in the scanning molecule counting method of detecting the light of the single particle, particles which are observation targets (observation target particles) are limited to the light emitting particles. Accordingly, when non-light emitting particles are to be observed, it is necessary to apply a light emitting label (fluorescence label, phosphorescence label or the like) to the particles which are the observation targets. However, an appropriate light emitting label is not always necessarily applied to the observation target particles. Furthermore, the observation target particles may be modified due to the application of the light emitting label.

An object of the invention is to provide a method of detecting non-light emitting target particles (detection target particles) in a sample solution for measurement using a scanning molecule counting method without applying a light emitting label, while suppressing influences of stray light and Raman scattering of water.

Means for solving the Problems

The inventors of the present invention have conducted intensive studies to achieve the object. As a result, the inventors of the present invention have found that in a scanning molecule counting method using an optical system of a confocal microscope or a multi-photon microscope, when light including substantially constant background light is detected from a light detection region of the optical system, the detected light intensity thereof almost does not vary in a case in which passing particles are sufficiently smaller than the light detection region, but in a case in which a relatively large particle passes, the particle blocks the light and the detected light intensity thereof is reduced. Using this phenomenon, the inventors of the present invention have developed an inverted scanning molecule counting method of counting non-light emitting molecules in a sample solution for measurement.

Furthermore, the inventors of the present invention have found that only with a conventional inverted scanning molecule counting method, it is very difficult to detect a molecule smaller than the light detection region, but when a non-light emitting particle in which the size of a molecule is relatively larger than the light detection region is used as an observation particle to bind the observation particle to target particle which is a detection target, and then the number of the observation particles is counted after removal of free observation particles outside the measurement system, it is possible to detect the observation particle even with the conventional inverted scanning molecule counting method. Furthermore, the inventors of the present invention have also found that using a solid phase carrier, free observation particles can be easily separated from the observation particles bound to the target particles, and has completed the invention.

That is, a target particle detection method according to the invention is as the following (1) to (14).

(1) A method of detecting a target particle, which is a method of detecting a non-light emitting particles using an optical system of a confocal microscope or a multi-photon microscope, the method including the steps of: (a) binding a non-light emitting observation particle and a solid phase carrier through a target particle which is a detection target in a solution or at an interface of the solution, thereby forming a complex; (b) removing observation particles in a free state from the solution after the step (a); (c) separating the observation particles from the complex after the step (b); (d) preparing a sample solution for measurement in which the separated observation particles are dispersed after the step (c); and (e) detecting, while moving the position of a light detection region of the optical system in the sample solution for measurement, light including substantially constant background light from the light detection region to generate light intensity data over time (time-series light intensity data), and individually detecting, as a signal indicating the presence of each of the observation particles, a reduction in the light intensity occurring when the observation particle enters the light detection region in the time-series light intensity data, thereby detecting the observation particle in the sample solution for measurement.

(2) The method of detecting target particle according to (1), in which an average outside diameter of the observation particles is 35% or greater of a diameter of the light detection region of the optical system.

(3) The method of detecting a target particle according to (1) or (2), in which the target particle is a nucleic acid molecules or a nucleic acid analog, and the separation of the observation particle in the step (c) is performed by denaturing the target particle in the complex.

(4) The method of detecting a target particle according to any one of (1) to (3), in which in the step (d), the solid phase carrier is separated and removed from the observation particles, and the sample solution for measurement in which the observation particles are dispersed is prepared.

(5) The method of detecting a target particle according to (1) or (2), in which the solid phase carrier is a particle in which an average outside diameter thereof is less than 35% of the diameter of the light detection region of the optical system, and (c') preparing a sample solution for measurement in which complexes including the observation particles, the target particles, and the solid phase carrier are dispersed after the process (b) is performed in place of the steps (c) and (d).

(6) The detection method of detecting a target particle according to any one of (1) to (5), in which the background light is fluorescence, phosphorescence, chemiluminescence, bioluminescence, or scattered light produced by the substance dispersed in the sample solution for measurement.

(7) The method of detecting a target particle according to any one of (1) to (5), in which the background light is illumination light.

(8) The method of detecting a target particle according to any one of (1) to (7), in which the position of the light detection region is moved at a speed higher than a diffusion moving speed of the single particle.

(9) The method of detecting a target particle according to any one of (1) to (8), in which the position of the light detection region is moved by changing an optical path of the optical system.

(10) The method of detecting a target particle according to any one of (1) to (9), in which the detection of the signal indicating the presence of the observation particles comprises determining one observation particle to have entered the light detection region when a signal having a light intensity lower than a predetermined threshold, measured from the intensity of the background light, is detected.

(11) The method of detecting a target particle according to any one of (1) to (10), in which the detection of the signal indicating the presence of the observation particles comprises smoothing the light intensity data over time (time-series light intensity data), and detecting a signal having an intensity lower than a predetermined threshold, measured from the intensity of the background light, and having a downward convex bell-shaped pulse form in the smoothed light intensity data over time (time-series light intensity data).

(12) The method of detecting a target particle according to any one of (1) to (11), in which in the step (e), further counting the number of the individually detected signals indicating the presence of the observed particles, thereby counting the number of the observation particles detected during the movement of the position of the light detection region.

(13) The method of detecting a target particle according to any one of (1) to (12), in which in the step (e), further determining a number density or a concentration of the observation particles in the sample solution for measurement based on the number of the detected observation particles.

(14) The method of detecting a target particle according to any one of (1) to (12), in which in the step (e), repeating the movement of the position of the light detection region, the detection of the light from the light detection region, and the detection of the signal indicating the presence of the observation particles until the number of the signals indicating the presence of the observation particles reaches a predetermined number, and determining the concentration of the observation particles in the sample solution for measurement based on a time required for the number of the signals to reach the predetermined number.

Effects of the Invention

Since the target particle detection method according to the invention uses a principle of a scanning molecule counting method, it is possible to detect a target particle even when the number density or the concentration of the target particles in a sample solution for measurement is significantly low. In addition, in the target particle detection method according to the invention, it is not necessary to apply a light emitting label to the target particle, and thus even a particle which is denatured when having an optical label applied thereto can be detected as a target particle. Furthermore, according to an aspect in which a reduction in background light is detected as a signal indicating the presence of a target particle in the presence of a certain amount of background light, stray light and Raman scattered light can be prevented from being erroneously detected as a signal of the target particle. Furthermore, in the target particle detection method according to the invention, a target particle is not directly detected, but indirectly detected by detecting an observation particle, and thus the detection is possible even when the a target particle is a molecule relatively smaller than a light detection region.

Brief Description of the Drawings

Fig. 1A is a schematic diagram of an internal structure of an optical analysis device for an inverted scanning molecule counting method.

Fig. 1B is a schematic diagram of a confocal volume (a light detection region of a confocal microscope).

Fig. 1C is a schematic diagram of a mechanism which moves a position of the light detection region in a sample solution by changing a direction of a mirror 7.

Fig. 1D is a schematic diagram of a mechanism which moves a position of the light detection region in the sample solution by moving a position in a horizontal direction of a microplate.

Fig. 2A is a schematic diagram illustrating a principle of the detection of the presence of a non-light emitting single particle in the inverted scanning molecule counting method.

Fig. 2B is a schematic diagram of a variation in the light intensity with time measured by the principle of Fig. 2A.

Fig. 2C is a diagram illustrating a principle of the reduction in the light amount detected when a non-light emitting single particle has entered the light detection region.

Fig. 2D is a diagram illustrating a relationship between a ratio of diameters of the light detection region and a single particle, and a proportion of the reduction in the detected light amount.

Fig. 3 is a diagram illustrating an aspect of processing procedures of the inverted scanning molecule counting method in a flowchart format.

Fig. 4A is a model diagram illustrating an aspect of the movement of a single particle when the particle crosses the light detection region while performing Brownian movement.

Fig. 4B is a model diagram illustrating an aspect of the movement of a single particle when the particle crosses the light detection region by moving the position of the light detection region in the sample solution at a speed higher than a diffusion moving speed of the particle.

Fig. 4C is a diagram illustrating an example of signal processing procedures of a detected signal in the processing procedures for detecting the presence of a single particle from measured time-series light intensity data (a variation in the photon counting with time) in accordance with the inverted scanning molecule counting method.

Fig. 5 is a diagram illustrating another aspect of processing procedures of the inverted scanning molecule counting method in a flowchart format.

Fig. 6A is a diagram illustrating still another aspect of processing procedures of the inverted scanning molecule counting method in a flowchart format.

Fig. 6B is a diagram illustrating the content of a process of modifying an analysis time interval t in accordance with particle detection circumstances in the aspect illustrated in Fig. 6A in a flowchart format.

Fig. 7 is a diagram illustrating results of the measurement of the number of peaks of each sample solution for measurement in Example 1.

Fig. 8 is a diagram illustrating results of the measurement of the number of peaks of each sample solution for measurement in Example 2.

Embodiments for carrying out the Invention

In a target particle detection method according to the invention (hereinafter, may be referred to as simply "detection method according to the invention"), a non-light emitting particle is detected using an inverted scanning molecule counting method using an optical system of a confocal microscope or a multi-photon microscope. The inverted scanning molecule counting method refers to a scanning molecule counting method of detecting light including substantially constant background light from a light detection region of the optical system, and of detecting the presence of a particle passing through the light detection region using a reduction in the light intensity detected from the light detection region as an index.

In the normal scanning molecule counting methods disclosed in Patent Documents 2 to 5, particles which are observation targets are light emitting particles, and non-light emitting particles cannot be detected. Accordingly, when non-light emitting particles are observation targets, it is necessary to apply a light emitting label such as a fluorescent label to the particle. However, according to the particles, it may be difficult to apply a light emitting label, or the particles may be denatured due to the application of the light emitting label. In addition, in a method in which light emitted from a particle is recognized as a signal indicating the presence of the particle, when a value on light intensity data is increased due to stray light, scattered light, or electrical noise of a light detector, the increase in the value may be erroneously recognized as a signal of the light emitting particle.

In general, when an optical measurement is performed using an optical system of a microscope, the measurement is rarely influenced by stray light and Raman scattering of water when background light is high. In addition, when an optical system of a confocal microscope or a multi-photon microscope is used, the resolution in an optical axis direction is higher than a normal optical microscope, and thus when a non-light emitting particle passes through a confocal volume, a reduction in the light intensity from the confocal volume is observed. That is, when the measurement is performed using the inverted scanning molecule counting method in the presence of sufficient background light, a target particle can be detected under an environment in which influences of stray light and Raman scattering of water are reduced. In addition, it is not necessary to perform labeling of the target particles by a light-emitting probe or the like.

In the invention and the specification of the present application, the "light detection region" of the optical system of the confocal microscope or the multi-photon microscope refers to a small region where light is detected in these microscopes. When illumination light is applied from an objective lens, the light detection region corresponds to a region where the illumination light is condensed. In the confocal microscope, such a region is determined from, particularly, a positional relationship between an objective lens and a pinhole.

In the invention and the specification of the present application, "particles which are dispersed in a solution and randomly move" refer to atoms, molecules, or particles such as aggregates thereof dispersed or dissolved in a solution (the particles may or may not emit light), and means particles which are not fixed to a substrate or the like and freely perform Brownian movement in a solution.

In the inverted scanning molecule counting method, as in the scanning molecule counting method, light is sequentially detected while the position of the light detection region is moved in a sample solution for measurement, that is, while the sample solution for measurement is scanned using the light detection region. In such a configuration, in a case in which the light from the light detection region includes substantially constant background light, when "a non-light emitting particle having a certain size with respect to the light detection region" enters the light detection region or when the light detection region which is moved in the sample solution for measurement includes the non-light emitting particle, the light intensity or the light amount of the background light reaching a light detection portion from the light detection region is reduced by the presence of the particle. Thus, in the inverted scanning molecule counting method, in the sequentially detected light, a reduction in the light intensity or the light amount of such background light is individually detected as a signal of the non-light emitting particle, and thus the presence of the particles is individually and sequentially detected one by one and various information related to the state of the particle in the solution is acquired. In the present specification, the "signal of the particle" refers to a signal indicating the presence of the particle unless specifically noted.

Hereinafter, first, the inverted scanning molecule counting method will be described.

<Configuration of Optical Analysis Device for Inverted Scanning Molecule Counting Method>

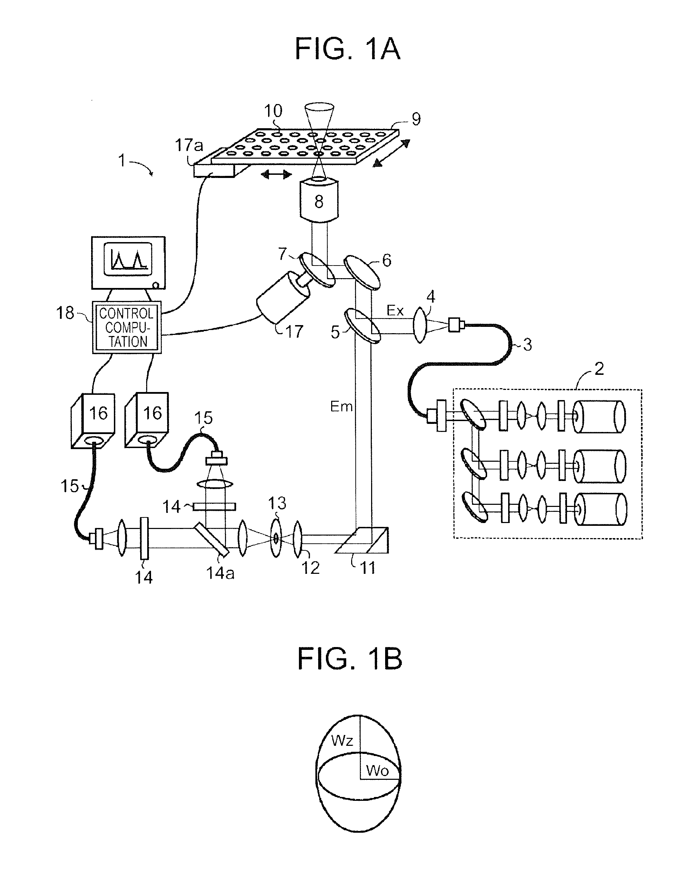

The inverted scanning molecule counting method can be realized through an optical analysis device having a basic configuration in which an optical system of a confocal microscope and a light detector are combined with each other to execute FCS, FIDA, and the like as schematically exemplified in Fig. 1A. Referring to Fig. 1A, an optical analysis device 1 has optical systems 2 to 17 and a computer 18 for controlling operations of the respective portions of the optical systems and acquiring and analyzing data. The optical systems of the optical analysis device 1 may be similar to optical systems of a normal confocal microscope, and laser light (Ex) radiated from the light source 2 and traveling in the single mode fiber 3 is emitted and radiated at an angle determined by unique NA at an emission end of the fiber and becomes parallel light by the collimator 4. The parallel light is reflected by the dichroic mirror 5 and the reflective mirrors 6 and 7 and enters the objective lens 8. Typically, the microplate 9 in which sample containers or wells 10 into which 1 $\mu$L to several tens of $\mu$L of sample solutions are dispensed, respectively, are arranged is disposed above the objective lens 8, and the laser light emitted from the objective lens 8 is focused in the sample solution in the sample container or the well 10 to form a high-light intensity region (excitation region). In the sample solution provided in the measurement, arbitrary light emitting substances generating background light may be dispersed or dissolved. In this case, when non-light emitting particles do not enter the excitation region, the light emitting substance is excited, and substantially constant light is emitted and becomes background light. Thus, when the non-light emitting particle enters the excitation region, the background light is reduced.

Thus, the light (Em) emitted from the excitation region passes through the objective lens 8 and the dichroic mirror 5 is reflected by the mirror 11, is condensed by the condenser lens 12, passes through the pinhole 13, transmits the barrier filter 14 (here, light components only in a specific wavelength band are selected), is introduced into the multimode fiber 15, reaches the light detector 16, and is converted into a time-series electrical signal. Then, the signal is input to the computer 18 to perform a process for detecting a single particle (a particle showing a behavior as one particle) in a manner to be described later. As known by those skilled in the art, in the above-described configuration, the pinhole 13 is disposed at a position conjugated with the focal position of the objective lens 8. Accordingly, as schematically illustrated in Fig. 1B, a focal region of laser light, that is, only light emitted from the excitation region passes through the pinhole 13, and light from a region other than the excitation region is blocked. In general, the focal region of laser light exemplified in Fig. 1B is a light detection region in the present optical analysis device having an effective volume of approximately 1 fL to 10 fL (Typically, the light intensity is spread in accordance with a Gaussian distribution having a peak at the center of the region. The effective volume is a volume of an approximate ellipsoid bordering a surface where the light intensity is $1/e^2$.), which is called confocal volume. In addition, in the invention, since a reduction in the light amount due to the presence of a single particle in background light composed of weak light from approximately several fluorescent dye molecules is detected, an ultrasensitive light detector capable of being used in photon counting is preferably used as the light detector 16. When light is detected through the photon counting, the light intensity thereof is measured in such a manner that the number of photons arriving at the light detector is sequentially measured for every predetermined unit time (BIN TIME) over a predetermined time. Accordingly, in this case, the time-series light intensity data is time-series photon count data. Furthermore, the stage (not shown) of the microscope may be provided with a stage position changing device 17a for moving the position in a horizontal direction of the microplate 9 to change a well 10 to be observed. The computer 18 may control the operation of the stage position changing device 17a. By virtue of such a configuration, it is possible to rapidly perform the measurement even when there is a plurality of samples.

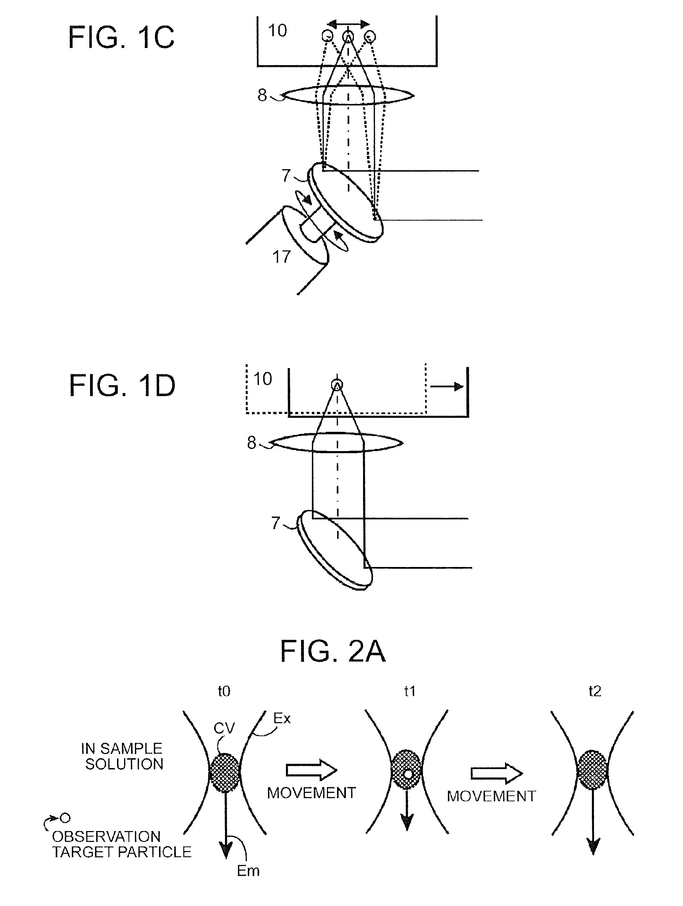

Furthermore, in the optical system of the above-described optical analysis device, a mechanism for performing scanning of the sample solution using the light detection region, that is, for moving the position of the focal region in the sample solution, that is, the light detection region is provided. As such a mechanism for moving the position of the light detection region, for example, as schematically exemplified in Fig. 1C, a mirror deflector 17 which changes the direction of the reflective mirror 7 (a type of moving the absolute position of the light detection region) may be employed. Such a mirror deflector 17 may be similar to a galvanomirror device equipped on a normal laser scanning microscope. Alternatively, as another aspect, as exemplified in Fig. 1D, the stage position changing device 17a may act to move the position in the horizontal direction of the container 10 (microplate 9) into which a sample solution has been injected, in order to move the relative position of the light detection region in the sample solution (a type of moving the absolute position of the sample solution). Even in either of the types, in order to attain a desired moving pattern of the position of the light detection region, the mirror deflector 17 or the stage position changing device 17a is driven in harmony with the light detection of the light detector 16 under the control of the computer 18. The moving track of the position of the light detection region may be arbitrarily selected from circular, elliptical, rectangular, linear and curvilinear ones, or a combination thereof (in the program in the computer 18, it may be designed so that various moving patterns can be selected). In addition, the type of moving the absolute position of the light detection region and the type of moving the absolute position of the sample solution may be combined to move the absolute position of the light detection region while moving the position of the sample solution. In this case, the light detection region passes through the same region within a short time, and thus the repeated detection of the same single particle is avoided. Otherwise, using the type of moving the absolute position of the light detection region, the light detection region may be purposefully allowed to repeatedly pass through the same region to periodically detect the same single particle a plurality of times to thus improve the accuracy of the signal. In this case, after the movement of the absolute position of the light detection region over a predetermined time, the position of the sample solution may be intermittently moved to repeatedly detect the same single particle, and the number of single particles to be detected may be increased. Although not illustrated in the drawing, the position of the light detection region may be moved in a vertical direction by moving the objective lens 8 or the stage up and down to three-dimensionally lay the track of the position of the light detection region out in the sample solution.

When light emitting substances generating background light emit light by multiple photon absorption, the above-described optical system is used as a multi-photon microscope. In that case, since the light is emitted only from the focal region (light detection region) of excitation light, the pinhole 13 may be removed. In addition, when light emitting substances generating background light emit light due to a chemiluminescence or bioluminescence phenomenon without excitation light, the optical systems 2 to 5 for generating excitation light may be omitted. When light emitting substances generating background light emit light due to phosphorescence or scattering, the above-described optical system of the confocal microscope is used as it is. Furthermore, in the device 1, as shown in the drawing, a plurality of excitation light sources 2 may be provided so that the wavelength of the excitation light can be appropriately selected according to the excitation wavelength of the light emitting substances. Similarly, a plurality of light detectors 16 may also be provided so that the detection can be separately performed according to the wavelength of the background light. The background light may be applied by illumination light. In that case, the sample solution is irradiated with transmitted illumination (Koehler illumination is also possible) from the upper side of the objective lens.

<Principle of Optical Analysis Technique of Inverted Scanning Molecule Counting Method>

Briefly speaking, in the inverted scanning molecule counting method, the presence of single particles is individually detected in such a manner that the position of the light detection region in a sample solution is moved using a scanning molecule counting method having a system which detects a shadow of the single particle, that is, in the presence of background light, and a reduction in the background light when the light detection region includes a non-light emitting single particle is detected as a signal of the single particle, and information related to the counting thereof or the concentration thereof in the sample solution is acquired.

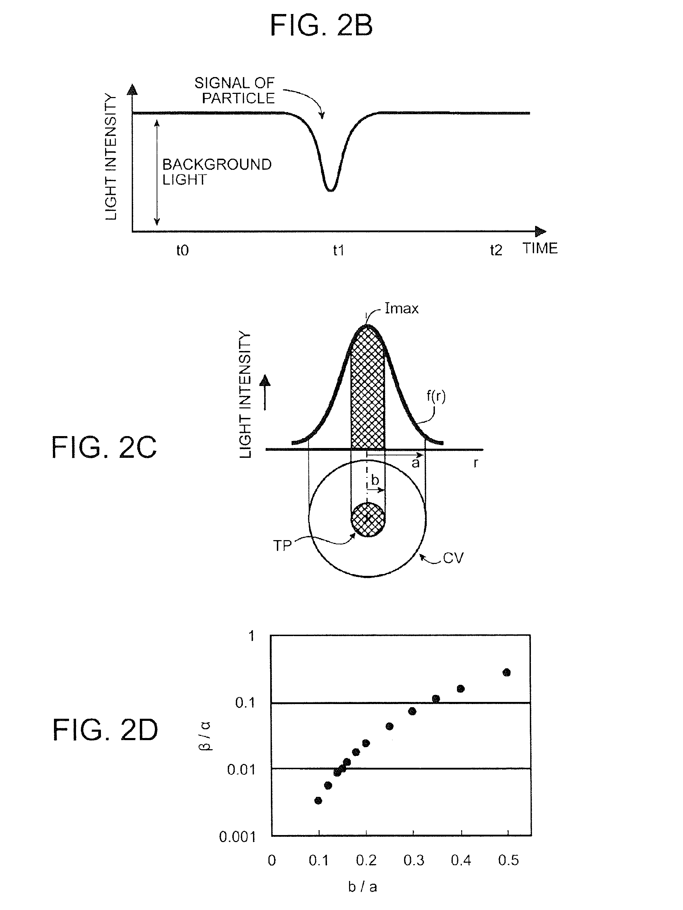

Specifically, as in a normal scanning molecule counting method, a mechanism for moving the position of the light detection region (mirror deflector 17) is driven to change the optical path, or the position in the horizontal direction of the container 10 (microplate 9) into which a sample solution has been injected is moved to detect light while moving the position of a light detection region CV in the sample solution as schematically illustrated in Fig. 2A, that is, while performing scanning the sample solution using the light detection region CV. As mentioned above, light emitting substances are dispersed in the sample solution and many light emitting substances are present in the light detection region CV. Accordingly, basically, the light from the light emitting substances is detected approximately uniformly during the movement of the light detection region CV (in the drawing, time t0 to time t2). However, when the light detection region CV passes through, during the movement, a region where one non-light emitting particle is present (t1), the volume of the region where the light emitting substances are present is reduced, and thus the total amount of the light emitted from the light emitting substances is reduced, and as illustrated in Fig. 2B, a significant reduction in the light intensity (Em) in a bell-shaped pulse form appears on the time-series light intensity data. Thus, by executing the movement of the position of the light detection region CV and the light detection and by detecting a significant reduction in the light intensity in a pulse form which appears during the execution as exemplified in Fig. 2B, that is, a signal indicating the presence of the single particle one by one, the single particles are individually detected, and the number of the single particles is counted to acquire information related to the number, the concentration, or the number density of the single particles present in the measured region.

The degree of the above-described reduction in the light intensity can be roughly calculated from a relationship between the diameter of the non-light emitting single particle and the diameter of the light detection region. Referring to Fig. 2C, typically, the light intensity distribution within the light detection region has a bell-shaped profile f(r) which has a maximum intensity Imax at the center thereof and is reduced in a radial direction r as shown by the solid line in Fig. 2C. Accordingly, using a radius a of the light detection region in which f(r) is approximately 0, a total amount $\alpha$ of the light emitted from the light detection region when no non-light emitting particle is present in the light detection region is given through the following expression:

$$\alpha = 4\pi \int r^2 f(r) dr \quad \text{[The interval of integration is 0 to a]}$$

$$...(1)$$

Meanwhile, when a non-light emitting single particle having a radius b enters the light detection region and is positioned at the center of the light detection region as illustrated in the lower part of Fig. 2C, the light emitting substances in that region are excluded, and thus the light amount corresponding to the oblique line region in the upper part of Fig. 2C is reduced. The light amount corresponding to the excluded light emitting substances, that is, a reduction amount $\beta$ is given through the following expression:

$$\beta = 4\pi \int r^2 f(r) dr \quad \text{[The interval of integration is 0 to b]}$$

$$...(2)$$

Thus, the proportion of the reduction in the light intensity can be roughly calculated through $\beta/\alpha$.

Here, when f(r) is a Gaussian function, $\alpha$ is 1, and a is 1, the following is satisfied:

$$f(r) = 0.684 \exp(-2r^2) \quad ...(3)$$

Fig. 2D is a diagram in which the proportion $\beta/\alpha$ of the reduction in the light intensity with respect to a radius ratio b/a is plotted using the expression (3). Referring to Fig. 2D, typically, when a fluctuation rate of the background light is approximately 1% and the proportion of the reduction in the light intensity caused by the single particle is 1% or less, the signal cannot be detected. Therefore, the ratio b/a of the radius of the single particle to the radius of the light detection region should be 0.15 or greater. In addition, when the proportion of the reduction in the light intensity caused by the single particle is 10% or greater, the ratio b/a of the radius of the single particle which is detectable to the radius of the light detection region is 0.35.

When a single particle to be observed is a quencher or an acceptor of fluorescence energy transfer, the single particle absorbs surrounding light (for example, 10 nm), and thus the radius of the single particle which is detectable may be shorter than the radius exemplified above.

<Processes of Inverted Scanning Molecule Counting Method>

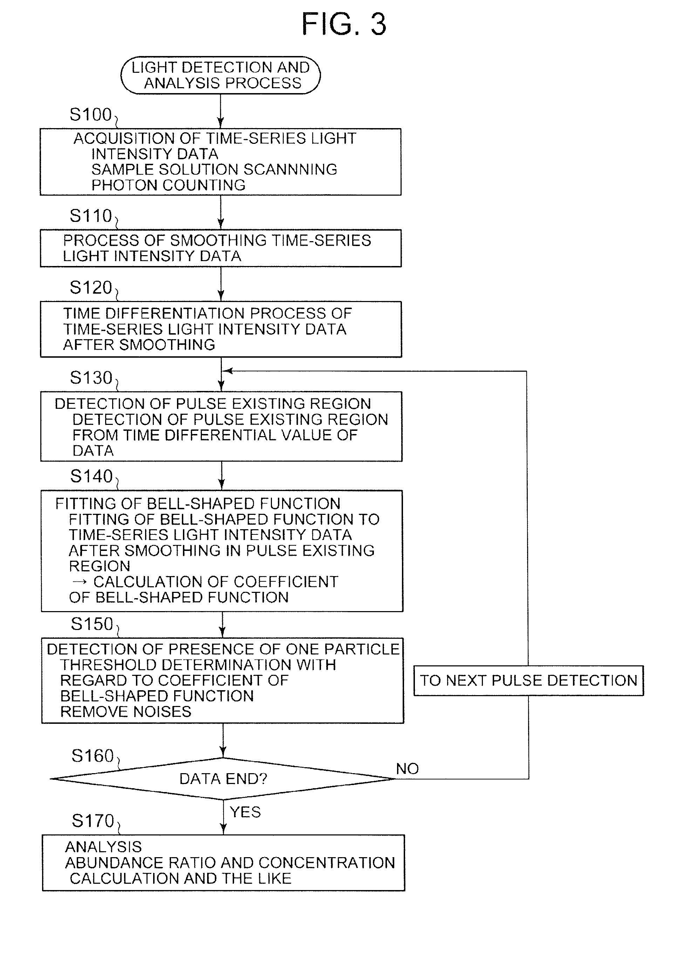

In the inverted scanning molecule counting method, the light intensity of a sample solution containing particles which are observation targets is measured, and then analyzed. Fig. 3 illustrates an aspect of the processing procedures of the inverted scanning molecule counting method in a flowchart format.

(Measurement of Light Intensity of Sample Solution: Step 100 of Fig. 3)

The measurement of the light intensity in the optical analysis using the inverted scanning molecule counting method may be executed in the same manner as in the process of measuring the light intensity in FCS or FIDA, except that the mirror deflector 17 or the stage position changing device 17a is driven during the measurement to move the position of the light detection region in the sample solution (scanning of the sample solution). In the operation process, typically, a sample solution is injected to a well 10 of the microplate 9 and the microplate 9 is placed on the stage of the microscope. Then, when a user inputs an instruction to the computer 18 to start the measurement, the computer 18 starts the irradiation of excitation light and the measurement of the light intensity in the light detection region in the sample solution in accordance with a program (procedures for moving the position of the light detection region in the sample solution and procedures for generating time-series light intensity data by detecting the light from the light detection region during the movement of the position of the light detection region) stored in a storage device (not shown). During such measurement, the mirror deflector 17 or the stage position changing device 17a drives the mirror 7 (galvanomirror) or the microplate 9 on the stage of the microscope under the control of the processing operation according to the program of the computer 18 to move the position of the light detection region in the well 10. Simultaneously with this, the light detector 16 converts the sequentially detected light into an electrical signal and transmits the electrical signal to the computer 18, and in the computer 18, time-series light intensity data is generated from the transmitted signal and is stored in an arbitrary manner. Typically, since the light detector 16 is an ultrasensitive light detector capable of detecting the arrival of a single photon, the time-series light intensity data may be time-series photon count data when the light is detected through the photon counting.

The moving speed of the position of the light detection region during the measurement of the light intensity may be arbitrarily, for example, a predetermined speed set experimentally or set to meet the purpose of the analysis. When acquiring, based on the number of the detected single particles, information related to the number density or the concentration thereof, the size or the volume of the region through which the light detection region has passes is required, and thus the position of the light detection region is moved in such a manner that the moving distance is grasped. Since the measurement results are easily analyzed when the measurement-elapsed time and the moving distance of the position of the light detection region are proportional to each other, the moving speed is basically preferably a constant speed, but is not limited thereto.

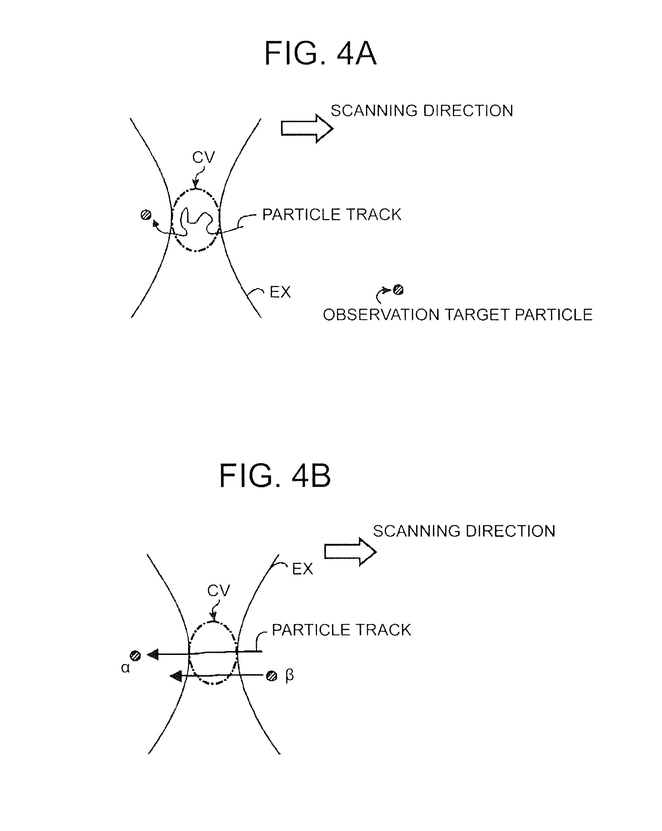

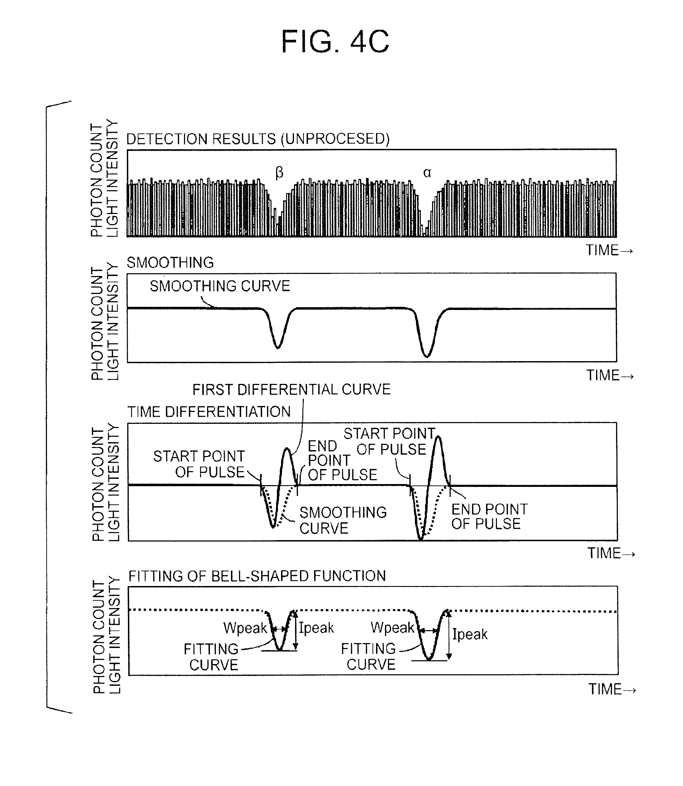

In order to quantitatively execute the individual detection of the single particles from the measured time-series light intensity data or the counting of the number of the single particles with high accuracy with regard to the moving speed of the position of the light detection region, such a moving speed is preferably set to a value higher than the moving speed of the single particle by random movement, that is, Brownian movement. Since the observation target particles of the inverted scanning molecule counting method are particles which are dispersed or dissolved in the solution and move freely and randomly, the positions thereof are moved by the Brownian movement with time. Accordingly, when the moving speed of the position of the light detection region is lower than that of the movement of the particle by the Brownian movement, as schematically illustrated in Fig. 4A, the particle is randomly moved within the region, and thus the light intensity randomly varies (as mentioned above, the excitation light intensity of the light detection region is reduced outward with a peak at the center of the region), and it becomes difficult to specify significant variations in the light intensity corresponding to the respective single particles. Accordingly, the moving speed of the position of the light detection region is preferably set to be higher than the average moving speed (diffusion moving speed) of the particles by the Brownian movement so that as illustrated in Fig. 4B, the particle approximately linearly crosses the light detection region, and thus the profile of the variations in the light intensity corresponding to the respective single particle becomes approximately uniform (when the single particle approximately linearly passes through the light detection region, the profile of the variations in the light intensity is approximately similar to the profile provided by inverting the excitation light intensity distribution) as exemplified in the top part of Fig. 4C in the time-series light intensity data, and the correspondence between the respective particle and the light intensity can be easily specified.

In addition, in the inverted scanning molecule counting method, when the light detection region passes through a position where a single particle is present, a reduction in the background light due to the presence of the single particle is detected to individually detect the single particle. However, when the single particle is randomly moved by performing Brownian movement in the solution, and enters and leaves the light detection region a plurality of times, a signal from one single particle, indicating the presence of the foregoing one single particle, is detected a plurality of times, and thus it becomes difficult to associate the detected signal with the presence of one single particle. Accordingly, as described above, the moving speed of the light detection region is set to be higher than the diffusion moving speed of the single particle, and thus it is possible to associate one single particle with one signal (indicating the presence of the single particle).

Specifically, from a relational expression of mean-square displacement:

$$(2Wo)^2 = 6D \cdot \Delta t \quad \ldots (4)$$

a time $\Delta t$ required when a particle having a diffusion coefficient D passes through a light detection region (confocal volume) having a radius Wo by Brownian movement is represented as follows:

$$\Delta t = (2Wo)^2 / 6D \quad \ldots (5)$$

Therefore, a moving speed Vdif of the particle by the Brownian movement (diffusion moving speed) is roughly represented as follows:

$$Vdif = 2Wo / \Delta t = 3D / Wo \quad \ldots (6)$$

Accordingly, the moving speed of the position of the light detection region may be set to a sufficiently higher value with reference to such Vdif. For example, in a case in which a diffusion coefficient D of an observation target particle is predicted to be approximately $2.0 \times 10^{-10} m^2/s$, when Wo is approximately 0.62 $\mu m$, Vdif is $1.0 \times 10^{-3} m/s$. Accordingly, the moving speed of the position of the light detection region may be set to 15 mm/s or the like, that is approximately more than 10 times higher than Vdif. When the diffusion coefficient of the observation target particle is unknown, various moving speeds of the position of the light detection region may be set and a preliminary test for finding a condition that a profile of a variation in the light intensity matches a predicted profile (typically, which is approximately similar to an excitation light intensity distribution) may be repeatedly executed to determine a preferred moving speed of the position of the light detection region.

(Light Intensity Analysis)

When the time-series light intensity data is obtained by the above-described process, various analyses such as the detection of the signals of the single particles, the counting of the single particles, and the calculation of the concentration are executed through a process according to the program stored in the storage device in the computer 18.

Whether one particle has entered the light detection region may be determined based on the shape of the time-series light intensity data by the above-described process. Typically, when a signal having a light intensity lower than a predetermined threshold, measured from the intensity of the background light, is detected, it may be determined that one single particle has entered the light detection region. More specifically, in general, in the time-series detected values of the light detection portion, that is, in the light intensity data, a signal indicating the presence of a single particle is shown as a signal having an intensity lower than a certain intensity and having a downward convex bell-shaped pulse form, and noise is shown as a signal having a high intensity or not having a bell-shaped. Accordingly, a signal having an intensity lower than a predetermined threshold, measured from the intensity of the background light, and having a downward convex bell-shaped pulse form on the time-series light intensity data may be detected as a signal indicating the presence of a single particle. The "predetermined threshold" can be set to an experimentally appropriate value.

Moreover, the light intensity obtained using the optical analysis device used in the inverted scanning molecule counting method is relatively low and is slightly increased or reduced. Such a slight increase or reduction in the light intensity deteriorates the accuracy of the detection of the signal indicating the presence of the single particle. Accordingly, after smoothing of the time-series light intensity data to process the data so that the slight increase or reduction in the light intensity can be ignored, a signal having an intensity lower than a predetermined threshold, measured from the intensity of the background light, and having a downward convex bell-shaped pulse form in the smoothed time-series light intensity data may be detected as a signal indicating the presence of the single particle.

In the inverted scanning molecule counting method, the number of signals may be counted to count the number of the single particles included in the light detection region (particle counting). In that case, by combining the number of the detected single particles and the moving amount of the position of the light detection region, information related to the number density or the concentration of the single particles identified in the sample solution is obtained. Specifically, for example, ratios of number densities or concentrations of a plurality of sample solutions, or ratios of relative number densities or concentrations with respect to a standard sample solution which is a reference in concentration or in number density may be calculated, or an absolute number density value or an absolute concentration value may be determined using the ratios of relative number densities or concentrations with respect to a standard sample solution which is a reference in concentration or in number density. Otherwise, when the total volume of the moving track of the position of the light detection region is specified by, for example, moving the position of the light detection region at a predetermined speed through an arbitrary method, the number density or the concentration of the single particles can be specifically calculated.

Hereinafter, further detailed description will be given.

(i) Individual Detection of Single Particle Signals

In the time-series light intensity data, when the track of one particle when the particle passes through the light detection region is approximately linear as shown in Fig. 4B, the variation in the light intensity of the signal corresponding to the particle has a profile reflecting the light intensity distribution in the light detection region (determined by the optical system) and having an approximately downward convex bell-shaped form (see the top part of Fig. 4C). Accordingly, in the scanning molecule counting method, basically, when a time width in which a reduction in the light intensity lower than an appropriately set threshold, measured from the background light, is continued is within a predetermined range, the signal having the profile of the foregoing reduction in the light intensity may be determined to correspond to the fact that one particle has passed through the light detection region, so that one particle is detected. A signal related to a case in which the time width in which the reduction in the light intensity lower than the threshold is continued is not within the predetermined range is determined as a signal of noise or foreign matter. In addition, in a case in which the light intensity distribution of the light detection region can be assumed as a downward convex Gaussian distribution from background light Ibg:

$$I=Ibg-A\cdot\exp(-2t^2/a^2) \quad \ldots(7)$$

when an intensity A and a width a calculated by fitting the expression (7) to the profile of the significant reduction in the light intensity (the profile which can be obviously determined to be not a fluctuation of the background light) are within a predetermined range, respectively, the profile of the light intensity may be determined to correspond to the fact that one particle has passed through the light detection region, so that one particle is detected. A signal related to a case in which the intensity A and the width a are not within the predetermined range, respectively, may be determined as a signal of noise or foreign matter so as to be ignored in the subsequent analysis and the like.

As an example of the method of collectively detecting the single particles from the time-series light intensity data, first, the time-series light intensity data (the top part "detection results (unprocessed)" of Fig. 4C) is subjected to a smoothing process (Step 110 of Fig. 3, the intermediate upper part "smoothing" of Fig. 4C). The light emitted from light emitting substances is stochastically emitted, and since the light intensity thereof is relatively low, a slight increase or reduction occurs and such a slight increase or reduction (fluctuation) in the light intensity deteriorates the accuracy of the detection of the signal indicating the presence of the single particle. The smoothing process is performed to ignore the slight increase or reduction on the data. The smoothing process may be performed using, for example, a moving-average method or the like. The parameters in performing the smoothing process, such as data points averaged at a time in the moving-average method and the number of moving averages, may be appropriately set in accordance with the moving speed (scanning speed) of the position of the light detection region and the BIN TIME upon acquisition of the light intensity data.

Next, in the time-series light intensity data after the smoothing process, a primary differential value with regard to the time of the time-series light intensity data after the smoothing process is computed (Step 120) to detect a time domain (pulse existing region) in which a signal having a significant pulse form (hereinafter, referred to as "pulse signal") is present. As exemplified in the intermediate lower part "time differentiation" of Fig. 4C, in the time differential value of the time-series light intensity data, the variation in the value increases at the time when the signal value is changed. Accordingly, the start point and the end point of a significant signal can be determined advantageously with reference to such a time differential value.

After that, a significant pulse signal is sequentially detected on the time-series light intensity data, and whether the detected signal is a signal corresponding to a single particle is determined. Specifically, first, on the time-series time differential value data of the time-series light intensity data, the start point and the end point of one pulse signal are searched and determined by sequentially referring to the time differential value to specify a pulse existing region (Step 130). When one pulse existing region is specified, the fitting of a downward convex bell-shaped function to the smoothed time-series light intensity data in the pulse existing region is performed (the lower part "bell-shaped function fitting" of Fig. 4C), and parameters of the pulse of the bell-shaped function, such as the peak intensity (the maximum reduction amount from the background light) Ipeak, the pulse width (full width at half maximum) Wpeak, and the correlation coefficient (of the least square method) in the fitting, are calculated (Step 140). In this regard, although the fitted bell-shaped function is typically a Gaussian function, it may be a Lorentz type function. It is determined whether the calculated parameters of the bell-shaped function are within the respective ranges assumed for the parameters of the bell-shaped profile drawn by the pulse signal detected when one light-emitting particle has passed the light detection region, that is, whether each of the peak intensity, the pulse width, and the correlation coefficient of the pulse is within a predetermined range, for example, whether the following conditions:

$$20 \ \mu sec < pulse \ width < 400 \ \mu sec$$

$$peak \ intensity > 4.0 [pc/10 \ \mu s] \qquad \ldots (A)$$

$$correlation \ coefficient > 0.95$$

are satisfied (Step 150). Thus, the signal, whose calculated parameters of the bell-shaped function are determined to be within the ranges assumed in the signal corresponding to one particle, is determined as a signal corresponding to one particle. In contrast, a pulse signal, whose calculated parameters of the bell-shaped function are not within the assumed ranges, is ignored as noise.

The search and the determination of the pulse signal in the processes of the above-described Steps 130 to 150 may be repetitively executed over the whole region of the time-series light intensity data (Step 160). Also, the process of individually detecting the signal of the light emitting particle from the time-series light intensity data is not limited to the above-described procedures and may be executed through an arbitrary method.

(ii) Determination of Particle Concentration

Furthermore, the number of particles may be determined by counting the number of the signals of the detected single particles (counting of particles). In addition, when the total volume of the region through which the light detection region has passed is calculated through an arbitrary method, the number density or the concentration of the particles in the sample solution is determined from the volume value thereof and the number of the particles (Step 170).

The total volume of the region through which the light detection region has passed may be theoretically calculated based on the wavelength of the excitation light or the detected light, the number of openings of the lens, and the adjusted state of the optical system. However, for example, it may be experimentally determined from the number of particles detected by subjecting a solution whose particle concentration is known (comparative solution) to the above-described light intensity measurement, particle detection, and counting under the same conditions as in the measurement of the sample solution to be examined, and from the concentration of the particles of the comparative solution. Specifically, for example, when the number of detected single particles in a comparative solution having a particle concentration C is N, a total volume Vt of the region through which the light detection region has passed is given through the following expression:

$$Vt = N/C \ \ldots (8)$$

In addition, a plurality of solutions having different single particle concentrations may be prepared as comparative solutions and may be subjected to the measurement so as to employ an average of the calculated Vt values as the total volume Vt of the region through which the light detection region has passed. When Vt is given, a particle concentration c of a sample solution whose single particle counting result is represented by n is given through the following expression:

$$c = n/Vt \ \ldots (9)$$

The volume of the light detection region and the total volume of the region through which the light detection region has passed may be given not using the above-described method but using an arbitrary method such as FCS and FIDA. In addition, in the device of this embodiment, regarding the moving pattern of the light detection region to be assumed, information of various standard particles on the relationship (expression (6)) between the concentration C and the number N of particles may be previously stored in the storage device of the computer 18 to use the appropriately stored relationship information when a user of the device carries out the detection of the single particle.

Thus, in the inverted scanning molecule counting method in which the particles are individually detected by scanning of the sample solution using the light detection region, the counting of the particles in the sample solution, the determination of the concentration, and the like can be performed according to the above-described processing procedures.

(4) Single Particle Detection Process of Detecting Given Number of Signals

In the above-described single particle detection process, the light is measured over a set time, and then on the obtained light intensity data, the signals of single particles are detected. That is, the number of the signals of single particles obtained during the arbitrarily set measurement time is counted. In that case, when the particle concentration in the sample solution is unknown and the light intensity is measured over a fixed measurement time, a sufficiently long measurement time is set for a case in which the particle concentration is low. When the particle concentration in the sample solution is high, the measurement of the light intensity is continued for more than a time required to determine the characteristics such as the concentration with permissible or satisfactory accuracy. In addition, when the particle concentration in the sample solution is lower than a concentration assumed by a tester and the set measurement time is insufficient, a large error occurs in the result. In this manner, the number of the signals of the detected single particles varies with the length of the set measurement time, and particularly, when the single particle concentration is low, unevenness in the value of the single particle concentration calculated from the number of the detected signals becomes large, and thus the accuracy may be reduced.

Accordingly, as another aspect of the counting of the particles, the measurement may be executed until the number of the signals of the single particles reaches an arbitrarily set number, so as to calculate the value of the single particle concentration based on the measurement time. That is, as another aspect of the single particle detection process, the measurement of the light intensity with the movement of the light detection region and the detection of the signals of the single particles may be repeatedly performed until the number of the signals reaches a predetermined number, and the time required for the number of the signals to reach the predetermined number may be measured to determine the particle concentration based on the time required for the number of the signals of the single particles to reach the predetermined number. According to such a configuration, when the particle concentration in the sample solution is high, the time required for the measurement of the light intensity is shortened, and when the particle concentration in the sample solution is low, the measurement of the light intensity can be continued until the number of particles attaining the accuracy required for the result (that is, particle concentration) is obtained. That is, the measurement time is optimized in accordance with the concentration of the single particles.

In addition, when the predetermined number to be reached by the number of the signals of the single particles is set to the number of particles attaining the accuracy required for the result, the time required for the number of the signals of the single particles to reach the predetermined number reflects the number of particles attaining the accuracy required for the result, and thus the concentration value of the particles determined based on the time is expected to have permissible or satisfactory accuracy. That is, when the predetermined number is set to the number for attaining the accuracy required for the result, unevenness in the time required to detect the predetermined number of single particles or in the arbitrary result derived therefrom is suppressed to be small, and thus the accuracy of the result can be made satisfactory.

(i) Basic Principle

The concentration value of the particles and the time required for the number of the signals to reach the predetermined number are associated with each other as follows. That is, in a sample solution having a particle concentration C, in a case in which the light detection region is moved at a scanning speed u over a time $\tau$, when a cross-section of the light detection region is represented by S, the number X of particle signals detected is represented as follows:

$$X = CSu\tau N_A \quad ...(10)$$

Here, $N_A$ is an Avogadro's number. Accordingly, when a time T is required for the number of the signals to reach a predetermined number XE, the particle concentration C is given as a function of the time T through the following expression:

$$C = XE/(STuN_A) \quad ...(11)$$

In the expression (11), a particle detection speed V per unit time is given through the following expression:

$$V=XE/T \quad ...(12)$$

based on the time T required for the number of the signals to reach the predetermined number XE and the number XE of the detected particles. Accordingly, the particle concentration C is represented as follows:

$$C=V/(SuN_A) \quad ...(13)$$

In this expression (13), the particle concentration C is primarily proportional to the detection speed V and the correspondence relationship between the particle concentration C and the detection speed V is easy to understand. Accordingly, in the actual test, the particle concentration C may be determined using the detection speed V.

(ii) Processing Operation Procedures

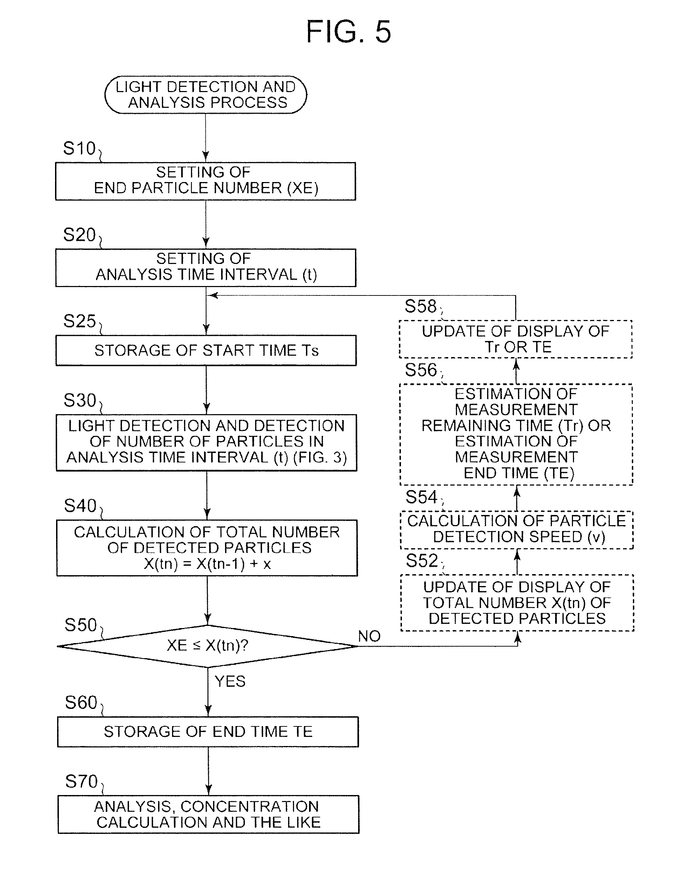

The single particle detection process of detecting a given number of signals may be executed according to, for example, the processing procedures illustrated in the flowchart of Fig. 5. In the example of Fig. 5, briefly speaking, a series of processes including the movement of the position of the light detection region, the detection of the light from the light detection region, the detection of the signals of the single particles, and the counting of the number of the detected particle signals is repeatedly executed every analysis time interval t (a predetermined time interval) until the number X of the detected particles reaches an end particle number XE (a predetermined number to be reached by the number of light emitting particles). It should be understood that the series of processes and configuration to be described below are realized by the processing operations of the computer 18.

(a) Initial Setting

Referring to Fig. 5, in the operation process, specifically, first, a sample solution is injected into a well 10 of the microplate 9 and the microplate 9 is placed on the stage of the microscope. Then, when a user inputs an instruction to the computer 18 to start processes for measurement of the light intensity and for particle detection and counting, the computer 18 performs the setting of the end particle number XE (Step 10) and the setting of the analysis time interval t (Step 20) as initial settings. The end particle number XE and the analysis time interval t may be arbitrarily set by a user. The end particle number XE can be appropriately determined with reference to results of a preliminary test using a solution whose particle concentration is known so that the accuracy required for the result value of the particle concentration can be attained. As the analysis time interval t, an arbitrary time interval which is sufficiently shorter than a time from after the start of the process to when the number (X) of particles reaches the end particle number (XE) may be appropriately determined with reference to the processing speed and the like of the device 1. In addition, regarding each of the end particle number XE and the analysis time interval t, a value predetermined with reference to the results of the preliminary test using the solution whose particle concentration is known may be stored in the device 1, and such a stored value may be used automatically or by selection of a user.

(b) Detection of Number of Particles

When the setting of the end particle number XE and the analysis time interval t is performed as described above; as described below, the measurement of the light intensity using the scanning molecule counting method over the analysis time interval t, the detection of particle signals from the measured light intensity data, and the detection of the number x of particles (Step 30); and a process of calculating a total number $X(t_n)$ of particles by accumulating the number x of particles detected in Step 30 (Step 40) are repeatedly executed every analysis time interval t until the total number $X(t_n)$ of particles reaches the end particle number XE (Step 50). Prior to the repeated execution of the processes of Steps 30 to 50, a start time Ts of the series of processes may be stored (Step 25).

The processes for light detection and for detection of the number of particles in Step 30 may be similar to the processes illustrated in Fig. 3. Briefly speaking, while the position of the light detection region is moved in the sample solution (scanning of the sample solution), the light intensity measurement is performed over the analysis time interval t, and then in the obtained time-series light intensity data in the analysis time interval t, the detection of signals indicating the presence of single particles and the counting of the number of the detection times are executed in the computer 18 through the process according to the program stored in the storage device.

Thus, when the number x of particles in the time-series light intensity data over the analysis time interval t is detected, the total number $X(t_n)$ of the detected light emitting particles is calculated as follows (Step 40 of Fig. 5) :

$$X(t_n) = X(t_{n-1}) + x \quad \ldots (14)$$

$X(t_{n-1})$ is the total number of the particles detected until the previous analysis time interval t, and its initial value is 0. Until the total number $X(t_n)$ of the detected light emitting particles reaches the end particle number XE, that is, until the following expression:

$$X(t_n) \geq XE \quad \ldots (15)$$

is established (Step 50), Steps 30 and 40 are repeated every analysis time interval t. When the expression (15) is established during the repetition of Steps 30 to 50, the processes for measurement of the light intensity of the sample solution and for particle detection and counting are ended. When the repetition of the processes of Steps 30 to 50 is ended, an end time TE may be stored (Step 60).

(c) Display of Number of Particles and Measurement End Time

A display such as a monitor of the computer 18 may display the total number $X(t_n)$ of the detected particles and/or the measurement end time TE or a measurement remaining time Tr during the period of time in which Steps 30 to 50 are repetitively executed every analysis time interval t (until the expression (15) is established). According to such a configuration, it is advantageous in that a user can predict when an executed measurement is ended by seeing those indications.

In a case in which the display is executed as described above, when the expression (15) is not established in the determination of Step 50 of Fig. 5, the respective processes shown by the dotted lines in Fig. 5 are executed. Specifically, first, the newest total number $X(t_n)$ of the detected particles calculated in Step 40 is displayed on the display (Step 52). When the repeated execution of Steps 30 to 50 has already been performed, the previous value of the total number $X(t_n)$ of the detected particles is updated. Next, in order to calculate the measurement end time TE or the measurement remaining time Tr, the speed v of the particle detection after the start of the processes of Steps 30 to 50 is calculated (Step 54). The speed v of the particle detection until the current time may be given the following expression:

$$v = X(t_n) / (T_p - T_s) \quad \ldots (16)$$

Here, $T_p$ represents a current time. Thus, using the particle detection speed v, the measurement remaining time Tr (a time to end the processes of Steps 30 to 50) is estimated as follows:

$$Tr = (XE - X(t_n)) / v \quad \ldots (17)$$

, and the measurement end time TE (a time at which the processes of Steps 30 to 50 are ended) is estimated as follows (Step 56):

$$TE = Tp + Tr \quad \ldots (18)$$

The estimated measurement end time TE or measurement remaining time Tr is displayed on the display (Step 58). When the repeated execution of Steps 30 to 50 has already been performed, the already displayed value is updated. In addition, when $X(t_n)$ is 0, Tr and TE may be displayed "unknown" without computation of the expression (17) or (18).

As described above, the above-described processes of Steps 30 to 50 of Fig. 5 are repeated every analysis time interval t. In this regard, the light intensity measurement of Step 100 of Fig. 3 may be continuously executed from the start to the end of the measurement even during the execution of the signal processing steps other than Step 100. That is, in the light detection and the detection of the number of particles, when one cycle of the light intensity measurement over the analysis time interval t is completed, the next cycle of the light intensity measurement over the analysis time interval t is executed continuously, and simultaneously with this, the processes for particle signal detection and counting from the light intensity data acquired over the analysis time interval t of the completed cycle are performed in the computer 18. Whereby, the detecting and counting of light-emitting particles will be achieved in real time.

(d) Analysis such as Concentration Calculation

Thus, when the number of particles reaches the end particle number, an analysis such as concentration calculation may be executed using the time T (=TE-$T_s$) until the number of particles reaches the end particle number or other information obtained from the detected signals of the particles (Step 70). As described above, for a particle concentration, a particle detection speed V is calculated from the time T to reach the end particle number and the end particle number XE using the expression (12), and the particle concentration is determined from the particle detection speed V using the relationship of the expression (13).

The cross-section S of the region through which the light detection region has passed in the expressions (10) to (13) may be theoretically calculated based on the wavelength of the excitation light or the detected light, the number of openings of the lens, and the adjusted state of the optical system. However, for example, it may be experimentally determined from the number of particles detected by subjecting a solution whose particle concentration is known (comparative solution) to the above-described light intensity measurement, particle detection, and counting under the same conditions as in the measurement of the sample solution to be examined, and from the concentration of the light emitting particles of the comparative solution. Specifically, for example, when the number of detected particles in the light intensity measurement executed over a time τo at a moving speed uo in a comparative solution having a particle concentration C is N, a cross-section S of the region through which the light detection region has passed is given through the following expression:

$$S=N/(C \cdot NA \cdot uo \cdot \tau o) \quad ...(19)$$

In addition, a plurality of solutions having different particle concentrations may be prepared as comparative solutions and may be subjected to the measurement so as to employ an average of the calculated S values as the cross-section S of the light detection region.

(e) Modified Examples of Processes for Measurement of Light Intensity of Sample Solution and for Particle Detection and Counting

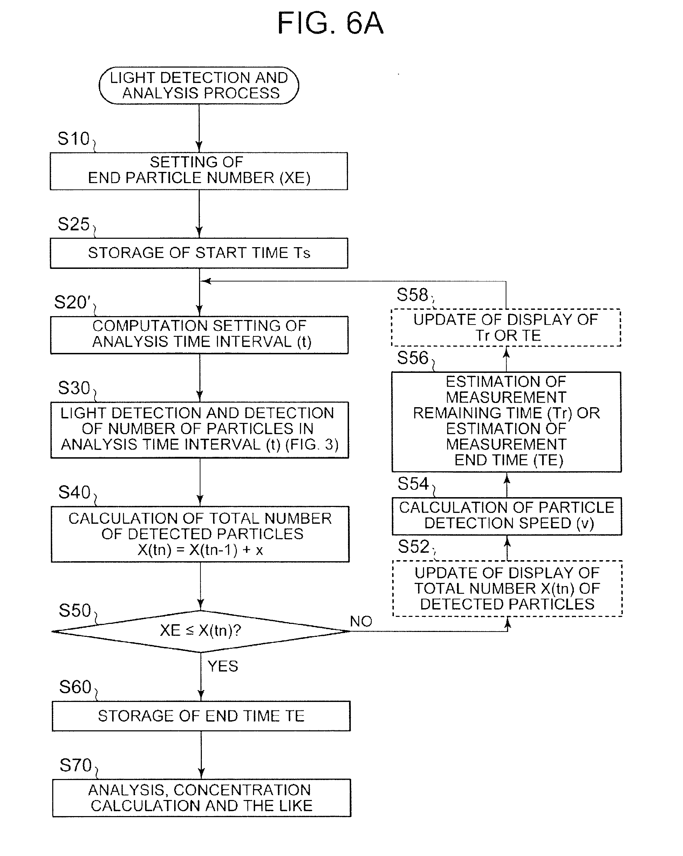

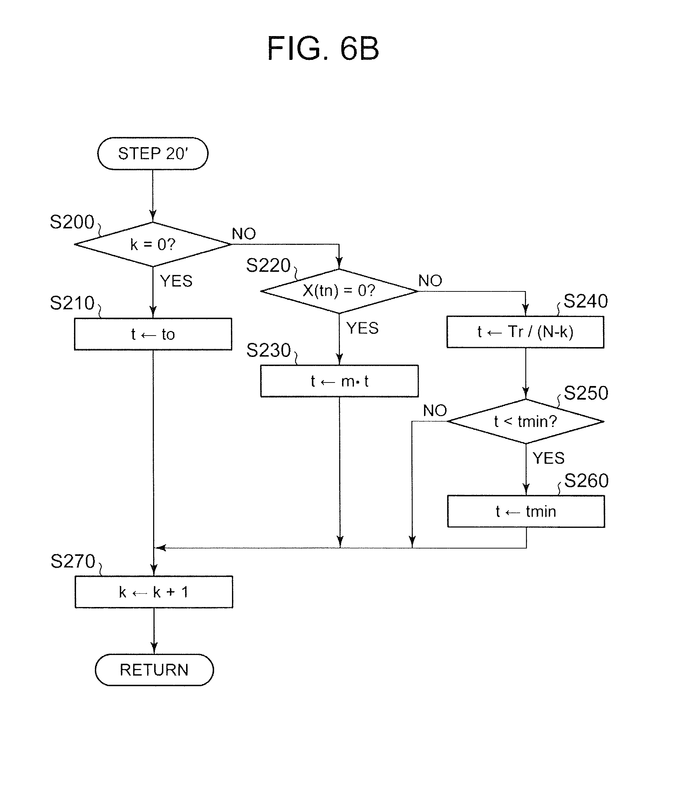

In the above-described processes for measurement of the light intensity of the sample solution and for light emitting particle detection and counting, as another aspect, the analysis time interval t may not be a fixed value, but may be modified in accordance with the light emitting particle detection circumstances. Fig. 6A illustrates processes for measurement of the light intensity of the sample solution and for particle detection and counting configured to include a process of modifying the analysis time interval t in accordance with the particle detection circumstances (Step 20') in a flowchart format, and Fig. 6B illustrates a process of computing the analysis time interval t in Step 20' in a flowchart format. In Fig. 6A, the same processes as those in Fig. 5 are denoted by the same step numbers.

Referring to Figs. 6A and 6B, in the processes of the figure, whenever the light intensity measurement over the analysis time interval t is completed, the analysis time interval t is modified (Step 20'). In addition, particularly, the processes in the examples illustrated in the figure are configured to execute the processing cycle of the light intensity measurement and the particle detection and counting only a predetermined number N of times (hereinafter, referred to as "the number of times of scheduled updating") in a single measurement from its start until the number of particles reaches the end particle number XE. Specifically, first, when the processes for measurement of the light intensity and for particle detection and counting are performed for the first time after the setting of the end particle number XE (Step 10) and the storing of the start time Ts (Step 25) as initial settings, that is, when the number k of times of execution of the processing cycle of the light intensity measurement and the particle detection and counting is 0, an initial value "to" which may be arbitrarily set is given as the analysis time interval t (see Steps 200 and 210 of Fig. 6B). The number k of times of execution of the processing cycle increases by one (Step 270), and the processes for measurement of the light intensity and for particle detection and counting over the analysis time interval t are executed (Steps 30 to 50) in the same manner as in the processes described in Fig. 5. Thus, when the number x of particles of the first cycle (=$X(t_1)$) is obtained, the particle detection speed v (Step 54) and the measurement remaining time Tr (Step 56) are sequentially calculated. As in the case of Fig. 5, the total number $X(t_n)$ of the detected particles and/or the measurement end time TE or the measurement remaining time Tr may be displayed on a display such as a monitor of the computer 18 (Steps 52 and 58). In addition, when the number of particles has reached the end particle number XE in the first processing cycle, the processes for measurement of the light intensity and for particle detection and counting are ended (Step 50).

After the first processing cycle, the modification of the analysis time interval t and the processing cycle of the light intensity measurement and the particle detection and counting (Steps 20' and 30 to 58) which are the same as in the case of Fig. 5 are repeated until the number of particles reaches the end particle number XE. In that case, in Step 20' in which the modification of the analysis time interval t is performed, first, it is determined whether the number $X(t_n)$ of the particles detected until that time is 0 (Step 220). When $X(t_n)$ is 0, the analysis time interval t in the last cycle may be increased m times (m represents a positive number which is 1 or greater). When $X(t_n)$ is greater than 0, the analysis time interval t is calculated through the following expression:

$$t=Tr/(N-k) \ \ ... (20)$$

using the measurement remaining time Tr, the number N of times of scheduled updating, and the number k of times of execution of the processing cycle (Step 240). As for the calculated analysis time interval t, the lower limit thereof may be set, and when the analysis time interval t is less than a lower limit value tmin, the analysis time interval t may be set to the lower limit value tmin (Steps 250 and 260). As described above, according to the aspect in which the analysis time interval t is modified, the measurement remaining time Tr reflects the detection circumstances of the particles which are observation targets in the sample solution, and therefore, the analysis time interval t is optimized in accordance with such particle detection circumstances.

Next, the target particle detection method of the invention using the inverted scanning molecule counting method will be described.

<Target Particle Detection Method>

In the detection method according to the invention, a non-light emitting particle is detected using an inverted scanning molecule counting method using an optical system of a confocal microscope or a multi-photon microscope. As described above, in the inverted scanning molecule counting method, particles having a relatively low concentration of not greater than pM-order can also be subjected to the measurement under circumstances in which molecules are dispersed. Therefore, using the detection method according to the invention, the number of target particles can be counted with high sensitivity even when the target particles which are analysis targets in a sample solution have a significantly low concentration.

Furthermore, in the detection method according to the invention, the number of target particles which are detection targets is not directly counted, but an observation particle which can be bound to the target particle and has such a size that the number thereof can be counted using the inverted scanning molecule counting method is used to detect the observation particle in a sample solution for measurement to indirectly detect the target particle. Therefore, even when the target particle has a molecule size smaller than the volume of a light detection region of the optical system (that is, when the particle passes through the light detection region, the light intensity detected from the light detection region is rarely reduced), the inverted scanning molecule counting method can be used, and it is possible to detect the target particle without applying a light emitting label while suppressing influences of stray light and Raman scattering of water.

Specifically, the detection method according to the invention has the following steps (a) to (e), and uses an optical system of a confocal microscope or a multi-photon microscope to detect a non-light emitting particle.

(a) a step of binding a non-light emitting observation particle and a solid phase carrier through a target particle which is a detection target in a solution or at an interface of the solution, thereby forming complexes;
(b) a step of removing the observation particles in a free state from the solution after the process (a);
(c) a step of separating the observation particle from the complex after the process (b);
(d) a step of preparing a sample solution for measurement in which the separated observation particles are dispersed after the process (c); and
(e) a step of detecting, while moving the position of a light detection region of the optical system in the sample solution for measurement, light including substantially constant background light from the light detection region to generate time-series light intensity data; and individually detecting, as a signal indicating the presence of each of the observation particles, a reduction in the light intensity occurring when the observation particles enter the light detection region in the time-series light intensity data, thereby detecting the observation particle in the sample solution for measurement.

The target particle which is a target for detection of the detection method according to the invention is a non-light emitting particle. The non-light emitting property is a property of emitting no light due to fluorescence, phosphorescence, chemiluminescence, bioluminescence, light scattering, or the like. The target particle may have the non-light emitting property with respect to background light and measurement light (light applied to the sample solution for measurement to detect the background light in the measurement using the inverted scanning molecule counting method), and may be a particle which emits fluorescence or the like when light having a wavelength other than those of the background light and the measurement light is applied thereto.

The size and the specific gravity of the target particles are not particularly limited. However, from the viewpoint of the fact that the effect of the invention, i.e., indirect counting of the number of the target particles using the observation particles, can be further exhibited, the target particles are preferably particles which are dispersed in a solution and randomly move. The target particles are more preferably particles in which the fluctuation rate of background light is 10% or less, and even more preferably particles in which the fluctuation rate of background light is 1% or less when passing through the light detection region of the optical system of the confocal microscope or the multi-photon microscope used in the measurement. It is very difficult to directly detect particles in which the fluctuation rate of background light is small, that is, 10% or less using the inverted scanning molecule counting method. However, the detection becomes possible when using the observation particles. Regarding the size of the target particles, for example, the average outside diameter is preferably less than 35%, and more preferably less than 30% of the diameter of the light detection region of the optical system.

In the detection method according to the invention, the target particles may be arbitrary particle and are not particularly limited, as long as these are non-light emitting particles. Examples thereof include biomolecules such as protein, peptide, nucleic acid, nucleic acid analog, lipid, sugar chain, amino acid and aggregates thereof, particulate biological objects such as virus and cell, and non-biological particles (such as atom, molecule, micelle, and metallic colloid). Furthermore, different kinds or the same kind of biomolecules may bind thereto. The nucleic acid may be DNA or RNA, or may be artificially amplified in the manner of cDNA. Examples of the nucleic acid analog include those in which side chains and the like of naturally-occurring nucleotides (nucleotides present in nature) such as DNA or RNA are modified with a functional group such as an amino group, and those labeled with proteins, low molecular weight compounds or the like. Specific examples thereof include bridged nucleic acids (BNA), nucleotides in which an oxygen atom at the 4' position of a naturally-occurring nucleotide is substituted with a sulfur atom, nucleotides in which a hydroxyl group at the 2' position of a naturally-occurring nucleotide is substituted with a methoxy group, hexitol nucleic acids (HNA), and peptide nucleic acids (PNA).

The target particles in the detection method according to the invention are preferably nucleic acid molecules or nucleic acid analogs. The nucleic acid molecules or nucleic acid analogs may be double-stranded nucleic acid molecules or single-stranded nucleic acid molecules. Specific examples thereof include nucleic acid molecules having a base sequence present in chromosomes of animal or plant or in genes of bacteria or viruses, and nucleic acid molecules having an artificially designed base sequence. Among these, preferable examples of the target particles include micro-RNA, siRNA, mRNA, hnRNA, genomic DNA, synthetic DNA obtained by PCR amplification or the like, and cDNA synthesized from RNA using reverse transcriptase.

Both of the observation particles and the solid phase carrier used in the detection method according to the invention have, on a surface thereof, a site specifically or non-specifically bound or adsorbed to the target particles (hereinafter, "target particle binding site"). In addition, the observation particles and the solid phase carrier are bound through the target particles. That is, the target particle binding site of the observation particles and the solid phase carrier is independently bound to a different site of one target particle.

For example, when the target particles are nucleic acid molecules or nucleic acid analogs, examples of the target particle binding site include oligonucleotides hybridizing with target particles, nucleic acid binding proteins (such as nucleic acid binding antibodies), and non-light emitting dye molecules bound to nucleic acids. The oligonucleotide may be DNA or RNA, may be artificially amplified in the manner of cDNA, or may be a substance a part or all of which includes a nucleotide chain or a nucleic acid analog which is capable of forming base pairs, similarly to natural nucleic acid bases. In addition, when the target particles are a protein, an antigen or an antibody to the target particles, a ligand or a receptor to the target particles, or the like can be used as the target particle binding site.

The target particle binding site of the observation particles and the solid phase carrier used in the invention may be non-specifically bound to the target particles, and is preferably specifically bound from the viewpoint of the accuracy of the detection and quantitation of the target particles. The target particle binding site which is specifically bound to the target particles may be preferentially bound to the target particles, rather than bound to other substances which are similar to the target particles in terms of physical or chemical properties, and may be bound to other substances other than the target particles. For example, when the target particles are nucleic acid molecules, the oligonucleotide used as a target particle binding site may have a base sequence completely complementary to the partial base sequence of the target particles, or a base sequence having a mismatch of one to several bases with the partial base sequence of the target particles.

The observation particles and the solid phase carrier used in the detection method according to the invention can be manufactured by, for example, immobilizing a substance having a target particle binding site (hereinafter, may be referred to as "substance to be bound to the target particles") to the surfaces of the particles and the solid phase carrier. The method of immobilizing the substance to be bound to the target particles to the surfaces of the observation particles and the solid phase carrier is not particularly limited, and may be a physical adsorption method, or a method for chemical binding to a functional group of the surfaces of the observation particles or the solid phase carrier. In the case of the chemical binding, binding can be performed through a method appropriate for each functional group. Examples thereof include an EDAC reaction, a reaction of binding a carboxylic acid to a amino group by previously mixing an EDC and a NHS, a reaction of crosslinking amino groups to each other using bipolar silica, and a reaction of binding an activated aldehyde group or tosyl group to a functional group in the substance to be bound to the target particles. When the surfaces of the observation particles or the solid phase carrier have no functional group, the surfaces may be previously covered with a functional group.

The observation particles and the solid phase carrier used in the detection method according to the invention are not limited to those directly having, on a surface thereof, the substance to be bound to the target particles. The substance to be bound to the target particles may be reversibly bound to the surfaces. For example, when the observation particles or the solid phase carrier have avidin on a surface thereof, the oligonucleotide hybridizing with the target particle may be labeled with biotin, the obtained biotin-labeled oligonucleotide may be bound to the avidin of the surfaces of the observation particles or the solid phase carrier, and these may be bound to the target particles to form complexes including the observation particles, the solid phase carrier, and the target particles.

The observation particles used in the detection method according to the invention are non-light emitting particles which are dispersed in a sample solution for measurement and randomly move. The observation particles are preferably particles in which the fluctuation rate of background light is greater than 10%, and more preferably particles in which the fluctuation rate of background light is 15% or greater when passing through the light detection region of the optical system of the confocal microscope or the multi-photon microscope used in the measurement. For example, regarding the size of the observation particles, the average outside diameter is preferably 35% or greater, and more preferably 50% or greater of the diameter of the light detection region. When the average outside diameter is 35% or greater of the diameter of the light detection region of the optical system, the observation particles which are dispersed in a sample solution for measurement and randomly move can be detected with high accuracy using the inverted scanning molecule counting method.

The material of the observation particles may have such a specific gravity that the observation particles are dispersed in a sample solution for measurement and can randomly move. Specific examples thereof include plastic particles such as polystyrene particles, latex particles, polyacrylamide resin particles, polymethylmethacrylate particles, polyethylene particles, polyvinyl chloride particles, polyamide particles, polyacrylate particles, and polyurethane particles; inorganic particles such as silica particles, ceramic particles, zirconia particles, silica-alumina particles, and glass particles; and agarose gel particles.

The solid phase carrier used in the detection method according to the invention is not particularly limited in shape, material, and the like, as long as it can be separated from the observation particles dispersed in the solution by solid-liquid separation. For example, the solid phase carrier may exist as particles which can be separated from the observation particles in accordance with the specific gravity, the presence or absence of magnetic property, or the like, or as a porous film, a container, a chip substrate, or the like.

The material of the solid phase carrier is not particularly limited, as long as it is insoluble in water or a solvent used in the measurement and the substance to be bound to the target particles can be immobilized to the surface of the solid phase carrier. Examples thereof include plastics such as natural rubber, polystyrene, polystyrene-divinylbenzene copolymer, styrene-butadiene copolymer, polyacrylic acid ester, and polymethacrylic acid ester; inorganic substances such as kaolin, carbon, carbon, activated carbon, glass, silica, alumina, and silica-alumina; natural organic polymers such as gelatin, liposome, and blood cell; and magnetic substances.

The solid phase carrier used in the invention is preferably magnetic particles, a silica membrane, a silica filter, a plastic plate, a plastic container, a glass container, and a chip substrate, and more preferably magnetic particles from the viewpoint of easy separation from free observation particles. When magnetic particles are used as the solid phase carrier, the observation particles are not magnetic particles.

The magnetic particles used as the solid phase carrier are not particularly limited, as long as magnetization can be easily carried out by magnetic induction. Examples of the magnetic particles include particles made of metals such as triiron tetroxide ($Fe_3O_4$), iron sesquioxide ($\gamma$-$Fe_2O_3$), various ferrites, iron, manganese, nickel, cobalt, and chromium; and particles made of alloys containing, cobalt, nickel, manganese, and the like. In addition, the magnetic particles may be particles including only a magnetic body, particles in which fine magnetic particles are contained in non-magnetic particles, or particles in which fine magnetic particles are immobilized to surfaces of non-magnetic particles. Examples of the non-magnetic particles include latex particles composed of a hydrophobic polymer, latex particles composed of a crosslinked hydrophilic polymer, gelatin, and liposome.

Examples of the hydrophobic polymer include polystyrene, polyacrylonitrile, polymethacrylonitrile, polymethyl methacrylate, polycapramide, and polythylene telephthalate. Latex particles composed of a copolymer of approximately two to four kinds of monomers may also be used.

Examples of the crosslinked hydrophilic polymer include polyacrylamide, polymethacrylamide, polyvinylpyrrolidone, polyvinyl alcohol, poly(2-oxyethyl acrylate), poly(2-oxyethyl methacrylate), poly(2,3-dioxypropylacrylate), poly(2,3-dioxypropylmethacrylate), polyethylene glycol methacrylate, and dextran.

In the detection method according to the invention, the particles used as the solid phase carrier may be particularly referred to as "separation particles". The size of the separation particles used in the invention is sufficiently small so that the separation particles cannot be detected using the inverted scanning molecule counting method. Particularly, the fluctuation rate of background light when the separation particles pass through the light detection region of the optical system is preferably 10% or less, and more preferably 1% or less. The reason is as follows: when the separation particles are not detected using the inverted scanning molecule counting method, the observation particles can be specifically detected by reducing noise even when free separation particles are dispersed in a sample solution for measurement in the measurement using the inverted scanning molecule counting method in the next process (e). For example, regarding the size of the separation particles, the average outside diameter is preferably less than 35%, and more preferably less than 30% of the diameter of the light detection region of the optical system.

Specifically, first, as the process (a), the observation particles and the solid phase carrier are bound to each other through the target particles to form complexes including the observation particles, the solid phase carrier, and the target particles. The binding reaction of the observation particles, the solid phase carrier, and the target particles can be caused in an appropriate solvent. The solvent is not particularly limited as long as it does not damage the target particles, the labeling particles, and the solid phase carrier. Typically, the solvent is an aqueous solution, but may be an organic solvent such as formamide or another arbitrary liquid. Specifically, the solvent can be appropriately selected and used from buffers which are generally used in the present technical field. Examples of the buffer include phosphate buffers such as phosphate buffered saline (PBS, ph 7.4) and tris buffers.

The observation particles, the solid phase carrier, and the target particles may simultaneously react with each other, or the respective binding reactions may be sequentially caused. For example, the observation particles and the target particles (in general, a test sample for examining whether the target particles are contained, and the same hereinafter) may be added to a solvent to prepare a solution containing the observation particles and the target particles, the observation particles and the target particles may be bound to each other in the solution, and then the solution may be brought into contact with the solid phase carrier (when separation particles are used, the separation particles are added to the solution) to bind the solid phase carrier to the target particles bound to the observation particles. In addition, a solution in which the target particles are added to a solvent may be brought into contact with the solid phase carrier (when separation particles are used, the separation particles are added to the solution) to bind the solid phase carrier and the target particles to each other, and then the observation particles may be added to the solvent to bind the observation particles to the target particles bound to the solid phase carrier. Regarding the expression "the solution is brought into contact with the solid phase carrier", for example, when the solid phase carrier is a porous film, the solution may pass through the porous film, when the solid phase carrier is a container, the solution may be injected into the container, and when the solid phase carrier is a chip substrate, the solution may be added dropwise to a surface of the chip substrate or the chip substrate may be dipped in the solution.

When the observation particles and the target particles, or the solid phase carrier and the target particles can be bound to each other only by allowing these to coexist with each other, by bringing a solution containing the observation particles and the target particles into contact with the solid phase carrier (when separation particles are used, the separation particles are added to the solution), and then if necessary, by incubating the solution for a predetermined time, complexes of the observation particles, the target particles, and the solid phase carrier can be formed in the solution.

When the target particles and the target particle binding site in the observation particles or in the solid phase carrier are nucleic acid molecules or nucleic acid analogs, the target particles and the target particle binding site are preferably allowed to meet together after denaturation of the nucleic acid molecules or the like in the solution. The expression "the nucleic acid molecules or the nucleic acid analogs are denatured" means dissociation of base pairs. For example, it means that double-stranded nucleic acid molecules are dissociated into single-stranded nucleic acid molecules. In a case in which the target particle binding site in the observation particles or in the solid phase carrier is an oligonucleotide containing an nucleic acid analog such as PNA, even when the target particles are double-stranded nucleic acid molecules, the target particle binding site and the target particles can be bound to each other without performing a special modification treatment.

Examples of the denaturation treatment include denaturation (thermal denaturation) by a high-temperature treatment and denaturation by a low-salt concentration treatment. Among these, thermal denaturation is preferably performed due to easy operation. Specifically, in the thermal denaturation, the nucleic acid molecules or the like in the solution can be denatured by subjecting the solution to a high-temperature treatment. In general, the modification can be carried out by keeping warm for approximately several seconds to two minutes at 90°C in the case of DNA or at 70°C in the case of RNA. However, the denaturation temperature varies with the length of the base of the target particle or the like, and is not limited to the foregoing temperatures when the denaturation can be carried out. The denaturation by the low-salt concentration treatment can be performed by, for example, adjusting the salt concentration of the solution to a sufficiently low level by dilution with purified water or the like.

After the denaturation has been carried out if necessary, the target particles in the solution and the target particle binding site in the observation particles or in the solid phase carrier are allowed to meet together to form complexes. When the thermal denaturation is performed, the target particles and the target particle binding site in the solution can be allowed to appropriately meet together by reducing, after the high-temperature treatment, the temperature of the solution to a temperature at which the target particles and the target particle binding site can specifically hybridize with each other (specific association condition). Preferably, the temperature of the solution including the target particles and the target particle binding site is reduced to a temperature of approximately the Tm value of a region having a base sequence complementary to the target particles in the target particle binding site $\pm 3°C$. In addition, when the denaturation by the low-salt concentration treatment is performed, the target particles and the target particle binding site in the solution can be allowed to appropriately associate together by increasing the salt concentration of the solution to a concentration at which the target particles and the target particle binding site in the observation particles or in the solid phase carrier can specifically hybridize with each other by addition of a salt solution or the like.

The temperature at which two single-stranded nucleic acid molecules can specifically hybridize with each other can be obtained from the melting curve of an assembly of the molecules. The melting curve can be obtained by, for example, changing the temperature of a solution containing only the molecules from high temperature to low temperature, and by measuring the absorbance or the fluorescence intensity of the solution. From the obtained melting curve, a temperature ranging from a temperature at which two modified single-stranded nucleic acid molecules start to form an assembly to a temperature at which almost all of the molecules form an assembly can be set as a temperature at which the molecules can specifically hybridize with each other. In place of the temperature, the salt concentration in the solution can also be changed from low concentration to high concentration to determine the melting curve in the same manner, and a concentration at which the two single-stranded nucleic acid molecules can specifically hybridize with each other can be obtained.

The specific association condition is different for each kind of the target particles and the target particle binding site and is experimentally determined. In general, the Tm value (melting temperature) can be used as a substitute.

For example, using primer/probe design software or the like which are widely used, the Tm value (a temperature at which 50% of double-stranded DNA is dissociated into single-stranded DNA) of a region hybridizing with the target particle can be calculated from base sequence information of the target particle binding site. A condition having its temperature close to the Tm value, for example, approximately within the Tm value $\pm 3°C$, can be set as the specific hybridization condition. The specific hybridization condition can be determined in further detail by experimentally obtaining the melting curve around the calculated Tm value.

In addition, in order to suppress non-specific hybridization, the temperature of the solution is preferably relatively slowly reduced in the formation of complexes. For example, the temperature of the solution can be reduced at a rate of temperature decrease of 0.05°C/sec or greater after the temperature of the solution is adjusted to 70°C or higher to denature the nucleic acid molecules.

In addition, in order to suppress non-specific hybridization, a surfactant, formamide, dimethylsulfoxide, urea or the like is preferably previously added to the solution. These compounds may be added singly or in combination of two or more kinds thereof. By adding these compounds, non-specific hybridization can be made unlikely under a relatively low temperature environment.

Next, as the process (b), observation particles in a free state are removed from the solution. That is, observation particles which are not bound to the solid phase carrier through the target particles are separated and removed from the complexes including the solid phase carrier, the target particles, and the observation particles. When the solid phase carrier is a porous film, a container, a chip substrate, or the like, the free observation particles can be separated and removed from the solid phase carrier by removing the liquid component from the solution prepared in the process (a). In addition, when the solid phase carrier is magnetic particles, for example, a magnet may be brought close to the container containing the solution prepared in the process (a) to converge the magnetic particles on a surface closest to the magnet in the container, and then the supernatant may be removed. When the solid phase carrier is particles heavier than the observation particles, the solution prepared in the process (a) may be subjected to a centrifugal separation process with such a centrifugal force that the solid phase carrier is precipitated, but the free observation particles are not precipitated, and the supernatant may be removed. When the solid phase carrier is particles sufficiently larger than the observation particles, the solution prepared in the process (a) may be filtered using a porous film of which the holes have such a size that the solid phase carrier cannot be permeated, but the observation particles can be permeated, and the solid phase carrier remaining on the surface of the porous film may be recovered.

Next, the observation particles are separated from the complexes as the process (c), and then a sample solution for measurement in which the separated observation particles are dispersed is prepared as the process (d). In order to detect particles using the inverted scanning molecule counting method, the particles are required to randomly move in the sample solution for measurement. When the solid phase carrier is a porous film, a container, a chip substrate, or the like, the observation particles in the solution can be dispersed by separating the observation particles from the complexes including the solid phase carrier. When the solid phase carrier is separation particles, the observation particles are also separated from the complexes including the solid phase carrier, and thus the degree of freedom of the movement of the observation particles in the solution increases compared to a state in which the observation particles are bound to the solid phase carrier, and thus the observation particles can be detected with high accuracy using the inverted scanning molecule counting method.

When the target particles are biomolecules, the observation particles are preferably separated from the solid phase carrier by decompositing the target particles. For example, in order to decompose the target particles, a nucleolytic enzyme treatment is performed when the target particles are nucleic acid, and a proteolytic enzyme treatment is performed when the target particles are a protein. Specifically, a solution containing a nucleolytic enzyme or a proteolytic enzyme is brought into contact with the solid phase carrier after the removal of the free observation particles in the process (b) to perform the enzyme treatment. Examples of the solvent of the solution containing these enzymes include the same solvents exemplified in the description of the process (a).

When the target particles are nucleic acid molecules or nucleic acid analogs, and the target particle binding site of the observation particles and the solid phase carrier is an oligonucleotide hybridizing with the target particle, the observation particles can be separated from the solid phase carrier by denaturing the nucleic acid molecules in the complexes including the observation particles, the target particles, and the solid phase carrier. For example, a solvent is added to the solid phase carrier after the removal of the free observation particles in the process (b) to prepare a solution containing the solid phase carrier, and then the solution is heated to 90°C to 100°C when the target particles are DNA, or to 70°C to 100°C when the target particles are RNA, whereby the observation particles bound to the solid phase carrier can be separated.

The sample solution for measurement in which the observation particles are dispersed can be prepared by separating the observation particles from the complexes including the solid phase carrier. However, the solid phase carrier is preferably not present in the light detection region in the sample solution for measurement since noise in the measurement of the inverted scanning molecule counting method can be further reduced. Accordingly, in the invention, the sample solution for measurement prepared in the process (d) is preferably a solution in which complexes including the solid phase carrier are separated and removed. Examples of the method of separating and removing the complexes including the solid phase carrier include methods similar to the method which can be used when removing, from the complexes including the solid phase carrier, the observation particles which are not bound to the solid phase carrier in the process (b). When the solid phase carrier is a container, the solution in the container can be made as a sample solution for measurement containing no complexes including the solid phase carrier mixed therein without performing a special separation process.

When separation particles which are sufficiently small and cannot thus be detected by the inverted scanning molecule counting method are used as the solid phase carrier, complexes (ternary complexes) including the observation particles, the target particles, and the separation particles may randomly move in the solution to the same extent as in the case of the free observation particles. Therefore, without separation from the separation particles, the ternary complexes can be provided as they are to the measurement using the inverted scanning molecule counting method. Specifically, a sample solution for measurement in which the ternary complexes are dispersed can be prepared by dispersing, in a solvent, the separation particles after the removal of the free observation particles in the process (b). Examples of the solvent include the same solvents exemplified in the description of the process (a).

Then, as the process (e), the observation particles in the sample solution for measurement prepared in the process (d) are calculated using the inverted scanning molecule counting method. The target particles are indirectly detected by detecting the observation particles.

Specifically, the sample solution for measurement is installed in the optical analysis device for the above-described inverted scanning molecule counting method, the position of the light detection region of the optical system in the sample solution for measurement is moved using the above-described method, and while the position of the light detection region in the sample solution for measurement is moved, the light including substantially constant background light is detected to generate time-series light intensity data. By individually detecting, as a signal indicating the presence of each of the observation particle, a reduction in the light intensity occurring when the observation particle has entered the light detection region in the time-series light intensity data, the observation particle is detected. The number of the observation particles detected during the movement of the position of the light detection region can also be counted by counting the number of the individually detected signals indicating the presence of the observation particles.

Information related to the number density or the concentration of the observation particles identified in the sample solution is obtained by combining the number of the detected observation particles and the moving amount of the position of the light detection region. Specifically, for example, ratios of number densities or concentrations of a plurality of sample solutions, or ratios of relative number densities or concentrations with respect to a standard sample solution for measurement which is a reference in concentration or in number density may be calculated, or an absolute number density value or an absolute concentration value may be determined using the ratios of relative number densities or concentrations with respect to a standard sample solution for measurement which is a reference in concentration or in number density.

Otherwise, when the total volume of the moving track of the position of the light detection region is specified by, for example, moving the position of the light detection region at a predetermined speed through an arbitrary method, the number density or the concentration of the observation particles can be specifically calculated. The number density or the concentration of the target particles in the test sample can also be determined based on the information, which has been obtained as described above.

In the measurement using the inverted scanning molecule counting method, the substantially constant background light to be included in the light from the light detection region may be illumination light produced by transmissive illumination or the like, or fluorescence, phosphorescence, chemiluminescence, bioluminescence, or scattered light produced by the substance dispersed in the sample solution for measurement. In this case, when no substance which emits or scatters light is dispersed in the solution used as the sample solution for measurement, the substance which emits or scatters light may be positively dissolved or dispersed in the solution. In addition, when the solution used as the sample solution for measurement emits autofluorescence, the autofluorescence may be used as the above-described background light.

For the background light, when a substance which emits or scatters light is dissolved or dispersed in the sample solution for measurement, the substance may be added to the sample solution for measurement before the measurement using the invented scanning molecule counting method. The substance is typically a fluorescent substance, but may be a substance which emits light by phosphorescence, chemiluminescence, bioluminescence, light scattering or the like. The fluorescent substance is not particularly limited as long as it is a substance which emits fluorescence by radiation of light having a specific wavelength, and an appropriate one selected from fluorescent dyes which are used in FCS, FIDA, and the like can be used.

Examples

Next, the invention will be described in further detail with examples and the like, but is not limited to the following examples.

[Example 1]

An oligonucleotide having a base sequence represented by Sequence No. 1 was used as target particles (target DNA), avidinated polystyrene beads were used as observation particles, biotinylated DNA probes 1 in which biotin was bound to an oligonucleotide having a base sequence represented by Sequence No. 2 were used as a "substance to be bound to the target particles" to be bound to surfaces of the avidinated polystyrene beads, avidinated magnetic beads were used as separation particles, and biotinylated DNA probes 2 in which biotin was bound to an oligonucleotide having a base sequence represented by Sequence No. 3 were used as a "substance to be bound to the target particles" to be bound to surfaces of the avidinated magnetic beads to perform the detection method according to the invention.

[Table 1]

| | Base Sequence |
|---|---|
| Sequence No. 1 | GACTGAATATAAACTTGTGGAGCCTGGGAAAGTCCCCTCAACT |
| Sequence No. 2 | CCACAAGTTTATATTCAGTC |
| Sequence No. 3 | AGTTGAGGGGACTTTCCCAGGC |

<Preparation of Avidinated Polystyrene Beads to which Biotinylated Probes were Bound>

A TES buffer (10 mM Tris-HCl, 100 mM EDTA, 1 M NaCl, pH 8.0) containing 160 fM of avidinated polystyrene beads (product name: sphero streptavidin SVP-40-5, manufactured by Sphero, diameter (average outside diameter): 4 $\mu$m) and a TES buffer containing 2.7 $\mu$M of 3'-biotinylated DNA probes 1 were mixed with each other at a ratio of 5:3 and were incubated while being stirred for 1 hour at room temperature to bind the 3'-biotinylated DNA probes 1 to the avidinated polystyrene beads.

The obtained DNA probe 1-modified polystyrene beads were washed 5 times through a centrifugal separation process, and thus unreacted 3'-biotinylated DNA probes 1 were removed.

<Preparation of Avidinated Magnetic Beads to which Biotinylated Probes were Bound>

A TES buffer containing 10 pM avidinated magnetic beads (product name: Dynabeads myone, manufactured by Invitrogen, diameter: 1 $\mu$m) and a TES buffer containing 10 $\mu$M of 5'-biotinylated DNA probes 2 were mixed with each other in equal amounts and were incubated while being stirred for 1 hour at room temperature to bind the 3'-biotinylated DNA probes 2 to the avidinated magnetic beads. The obtained DNA probe 2-modified magnetic beads were washed 5 times through a centrifugal separation process, and thus unreacted 3'-biotinylated DNA probes 2 were removed.

<Steps (a), (b), and (c')>

DNA probe 1-modified polystyrene beads, DNA probe 2-modified magnetic beads, a TES buffer, and various concentrations (0, 0.5, 1, 5, 10, 50 fM) of target DNA were mixed at a ratio of 1:5:3:1 and were incubated while being stirred for 24 hours at room temperature. The resulting solution was brought close to a magnet to cause precipitation, and the supernatant was removed. Then, a TES buffer was added to reach the original volume in order to suspend the precipitates. This solution and a 20 vol% polyethylene glycol solution containing 6 nM ATTO (registered trade name) 488 were mixed with each other in equal amounts to prepare a sample solution for measurement.

<Step (e)>

Complexes including the avidinated polystyrene beads in the prepared sample solution for measurement were detected using an inverted scanning molecule counting method. Specifically, in the measurement, time-series photon count data regarding each of the above-described sample solutions for measurement was acquired using a single molecule fluorescence measuring device MF20 (manufactured by Olympus Corporation) provided with an optical system of a confocal fluorescence microscope and a photon counting system. 488 nm laser light of 50 $\mu$W was used as excitation light and light having a wavelength band of 510 nm to 560 nm was measured using a bandpass filter 535BP to generate time-series light intensity data. A light detection region in the sample solution for measurement was designed to have a diameter of 4 $\mu$m and was rotated at a moving speed (scanning speed) of 9,000 rpm (67.5 mm/sec) to detect a signal obtained from an avalanche photodiode (APD) for every BIN TIME of 10 $\mu$sec, and the measurement was performed in a plate continuous measurement mode for 60 seconds. Each sample solution for measurement was subjected to the measurement 3 times to evaluate unevenness. Time-series data obtained by the measurement was subjected to smoothing with an algorithm of Savinzky-Golay, and then detection of peaks was performed by differentiation. Among regions regarded as peaks, regions capable of being approximated to a Gaussian function were extracted as signals to calculate the number of peaks.

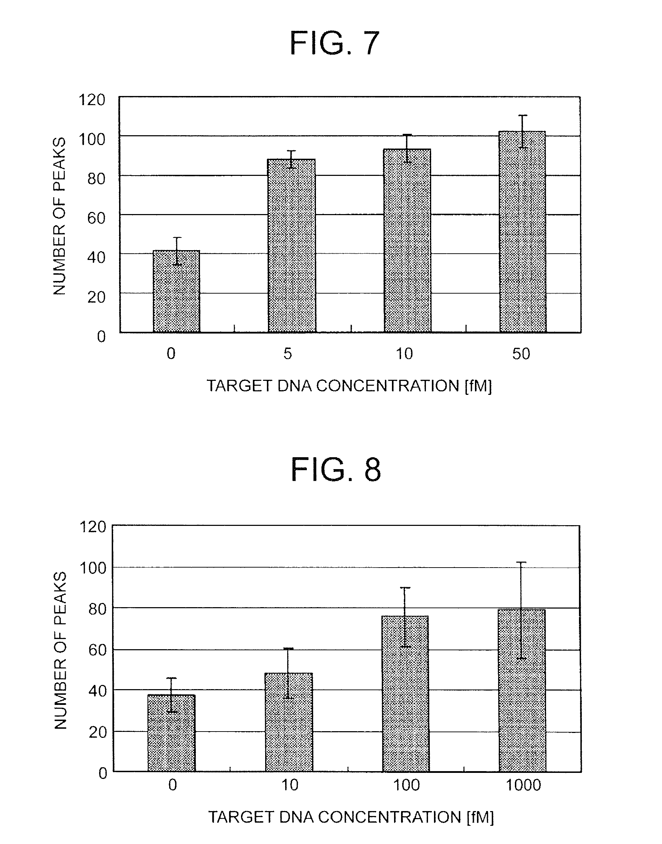

The number of peaks obtained as a result of the measurement regarding each sample solution for measurement is shown in Fig. 7. An increase in the number of peaks depending on the concentration of the target DNA was observed. Here, in this example, observation particles in which the average outside diameter is 100% of the diameter of the light detection region and observation particles in which the average outside diameter is 25% of the diameter of the light detection region are used. Accordingly, from the results, it is obvious that when observation particles in which the average outside diameter is 100% or greater of the diameter of the light detection region and observation particles in which the average outside diameter is 25% or less of the diameter of the light detection region are used, the target particle can be detected using the detection method according to the invention. Furthermore, since the range of an average number of peaks $\pm$1SD at a target DNA concentration of 5 fM did not overlap the range of an average $\pm$1SD at 0 M, it was obvious that through the detection method of this example, the concentration of the target particles can be detected with a detection limit of 5 fM.

[Example 2]

An oligonucleotide having a base sequence represented by Sequence No. 1 was used as target particles (target DNA), DNA-modified polystyrene beads in which an oligonucleotide (probe DNA 3) having a base sequence represented by Sequence No. 4 was immobilized to a surface were used as observation particles, avidinated magnetic beads were used as separation particles, and biotinylated D-array number NA probes 2 in which biotin was bound to an oligonucleotide having a base sequence represented by Sequence No. 3 were used as a "substance to be bound to the target particles" to be bound to surfaces of the avidinated magnetic beads to perform the detection method according to the invention.

[Table 2]

| | Base Sequence |
|---|---|
| Sequence No. 4 | CCACAAGTTTATATTCAGTCGGGGG |

<Preparation of DNA-Modified Polystyrene Beads>

A solution containing 100 μM of probe DNA 3 and a solution containing 100 μM of complementary strand DNA corresponding thereto were mixed with each other in equal amounts. Using a thermal cycler, the temperature of the obtained mixture was increased to 98°C, and then reduced to 20°C at 0.1°C/min to subject the DNA to annealing. Next, the solution containing the double-stranded DNA formed by annealing was diluted to 1/10 using ultrapure water. The solution and 50 mg/mL epoxy group-modified polystyrene beads (product name: micromer (registered trade name) epoxy, manufactured by Micromod, diameter: 4 μm) were mixed with each other in equal amounts, and were shaken for 24 hours at 50°C to react the amino group of guanine protruding from the probe DNA 3 with the epoxy group of the polystyrene beads so that the groups were bound to each other. Thus, double-stranded DNA-modified polystyrene beads were obtained. This reaction solution was subjected to a centrifugal separation process for 5 minutes at 15,000 rpm and the double-stranded DNA-modified polystyrene beads were precipitated and recovered by removing the supernatant. The recovered beads were subjected to a centrifugal separation process for 5 minutes at 15,000 rpm in 2.5 M KCl, and then washed by removing the supernatant. Next, the same washing operation was performed in 0.15 M NaOH, and subsequently in ultrapure water to dissociate the double-stranded DNA of the surfaces of the polystyrene beads into single-stranded DNA. Finally, the beads recovered as precipitates by the centrifugal separation process were suspended in a TES buffer, and thus 1.2 pM DNA-modified polystyrene beads in which single-stranded probe DNA 3 was modified on surfaces thereof were obtained.

<Steps (a), (b), (c), and (d)>

The prepared 1.2 pM DNA-modified polystyrene beads, 100 nM biotinylated DNA probes 2 used in Example 1, a TES buffer, and various concentrations (0, 0.1, 1, 10 pM) of target DNA used in Example 1 were mixed at a ratio of 1:1:7:1. The temperature of the resulting mixture was increased to 98°C using a thermal cycler, and then reduced to 20°C at 0.1°C/min to subject the DNA to annealing. Next, the solution containing the double-stranded DNA formed by annealing and avidinated magnetic beads (product name: Bio-Estapor Microspheres-BE-M08/0, 3, manufactured by Millipore) were mixed with each other in equal amounts and shaken for 1 hour at room temperature to crosslink the DNA-modified polystyrene beads with the avidinated magnetic beads through avidin-biotin binding. This solution was brought close to a magnet to cause precipitation, and the supernatant was removed. Then, a TES buffer was added to reach the original volume in order to suspend the precipitates. Thereafter, this solution was heated to 95°C and brought close to a magnet to cause precipitation, and the supernatant was recovered to obtain a solution containing the DNA-modified polystyrene beads separated from the avidinated magnetic beads. This solution and a 20 vol% polyethylene glycol solution containing 6 nM ATTO (registered trade name) 488 were mixed with each other in equal amounts to prepare a sample solution for measurement.

<Step (e)>

The DNA-modified polystyrene beads in the prepared sample solution for measurement were detected using the inverted scanning molecule counting method. Specifically, time-series data was obtained in the same manner as in Example 1, except that the measurement time was 100 seconds in a plate continuous measurement mode. The time-series data obtained by the measurement was subjected to smoothing with the algorithm of Savinzky-Golay, and then detection of peaks was performed by differentiation. Among regions regarded as peaks, the number of peaks with an odds ratio of 5.6 or greater was calculated.

The number of peaks obtained as a result of the measurement regarding each sample solution for measurement is shown in Fig. 8. The calculated average number of peaks ±1SD of each sample solution for measurement was $37\pm8.3$ at a target DNA concentration of 0 M, $48\pm12.0$ at 10 fM, $76\pm14.2$ at 100 fM, and $79\pm23.7$ at 1 pM. The number of peaks increased depending on the concentration of the target DNA and was saturated at 100 pM. In addition, since the range of an average number of peaks ±1SD at 100 fM or 1 pM did not overlap an average ±1SD at 0 M, it was obvious that through the detection method of this example, the concentration of the target particles can be detected with a detection limit of 100 fM.

Industrial Applicability

According to the detection method according to the invention, target particles present at an extremely low concentration in a test sample can be detected through an inverted scanning molecule counting method, and thus the detection method according to the invention can be used in fields such as analysis and examination of a sample in which the concentration of an analysis target substance such as a clinical specimen is low.

Description of Reference Numerals

1: optical analysis device (confocal microscope)
2: light source
3: single mode optical fiber
4: collimator lens
5: dichroic mirror
6, 7, 11: reflective mirror
8: objective lens
9: microplate
10: well (sample solution container)
12: condenser lens
13: pinhole
14: barrier filter
14a: dichroic mirror or polarization beam splitter
15: multimode optical fiber
16: light detector
17: mirror deflector
17a: stage position changing device
18: computer

**Claims**

1. A method of detecting a particle, which is a method of detecting a non-light emitting particle using an optical system of a confocal microscope or a multi-photon microscope, the method comprising the steps of:

   (a) binding a non-light emitting observation particle and a solid phase carrier through a target particle which is a detection target in a solution or at an interface of the solution, thereby forming a complex;
   (b) removing observation particles in a free state from the solution after the step (a);
   (c) separating the observation particle from the complex after the step (b);
   (d) preparing a sample solution for measurement in which the separated observation particles are dispersed after the step (c); and
   (e) detecting, while moving the position of a light detection region of the optical system in the sample solution for measurement, light including substantially constant background light from the light detection region to generate light intensity data over time, and individually detecting, as a signal indicating the presence of each of the observation particles, a reduction in the light intensity occurring when the observation particle enters the light detection region in the light intensity data over time, thereby detecting the observation particle in the sample solution for measurement.

2. The method of detecting a target particle according to claim 1,
   wherein an average outside diameter of the observation particles is 35% or greater of a diameter of the light detection region of the optical system.

3. The method of detecting a target particle according to claim 1 or 2,
   wherein the target particle is a nucleic acid molecule or a nucleic acid analog, and
   wherein the separation of the observation particle in the step (c) is performed by denaturing the target particle in the complex.

4. The method of detecting a target particle according to any one of claims 1 to 3,
   wherein in the step (d), the solid phase carrier is separated and removed from the observation particles, and the sample solution for measurement in which the observation particles are dispersed is prepared.

5. The method of detecting a target particle according to claim 1 or 2,
   wherein the solid phase carrier is a particle in which an average outside diameter thereof is less than 35% of the diameter of the light detection region of the optical system, and
   wherein (c') preparing a sample solution for measurement in which complexes including the observation particles, the target particles, and the solid phase carrier are dispersed after the process (b) is performed in place of the steps (c) and (d).

6. The method of detecting a target particle according to any one of claims 1 to 5,
   wherein the background light is fluorescence, phosphorescence, chemiluminescence, bioluminescence, or scattered light produced by the substance dispersed in the sample solution for measurement.

7. The method of detecting a target particle according to any one of claims 1 to 5,
   wherein the background light is illumination light.

8. The method of detecting a target particle according to any one of claims 1 to 7,
   wherein the position of the light detection region is moved at a speed higher than a diffusion moving speed of the single particle.

9. The method of detecting a target particle according to any one of claims 1 to 8,
wherein the position of the light detection region is moved by changing an optical path of the optical system.

10. The method of detecting a target particle according to any one of claims 1 to 9,
wherein the detection of the signal indicating the presence of the observation particle comprises;
determining one observation particle to have entered the light detection region when a signal having a light intensity lower than a predetermined threshold, measured from the intensity of the background light, is detected.

11. The method of detecting a target particle according to any one of claims 1 to 10,
wherein the detection of the signal indicating the presence of the observation particle comprises;
smoothing the light intensity data over time; and detecting a signal having an intensity lower than a predetermined threshold, measured from the intensity of the background light, and having a downward convex bell-shaped pulse form in the smoothed light intensity data over time.

12. The method of detecting a target particle according to any one of claims 1 to 11,
wherein in the step (e), further counting the number of the individually detected signals indicating the presence of the observation particles, thereby counting the number of the observation particles detected during the movement of the position of the light detection region.

13. The method of detecting a target particle according to any one of claims 1 to 12,
wherein in the step (e), further determining a number density or a concentration of the observation particles in the sample solution for measurement based on the number of the detected observation particles.

14. The method of detecting a target particle according to any one of claims 1 to 12,
wherein in the step (e), repeating the movement of the position of the light detection region, the detection of the light from the light detection region, and the detection of the signal indicating the presence of the observation particles until the number of the signals indicating the presence of the observation particles reaches a predetermined number, and
determining the concentration of the observation particles in the sample solution for measurement based on a time required for the number of the signals to reach the predetermined number.

## FIG. 1A

## FIG. 1B

FIG. 1C

10

8

7

17

FIG. 1D

10

8

7

FIG. 2A

t0                          t1                          t2

CV          Ex

IN SAMPLE
SOLUTION

MOVEMENT                  MOVEMENT

○
OBSERVATION
TARGET PARTICLE

Em

# FIG. 2B

# FIG. 2C

# FIG. 2D

# FIG. 3

```
       ╭─────────────────────╮
       │ LIGHT DETECTION AND │
       │  ANALYSIS PROCESS   │
       ╰─────────────────────╯
                 │
 S100            ▼
┌──────────────────────────────────┐
│ ACQUISITION OF TIME-SERIES LIGHT │
│   INTENSITY DATA                 │
│   SAMPLE SOLUTION SCANNNING      │
│   PHOTON COUNTING                │
└──────────────────────────────────┘
                 │
 S110            ▼
┌──────────────────────────────────┐
│ PROCESS OF SMOOTHING TIME-SERIES │
│ LIGHT INTENSITY DATA             │
└──────────────────────────────────┘
                 │
 S120            ▼
┌──────────────────────────────────┐
│ TIME DIFFERENTIATION PROCESS OF  │
│ TIME-SERIES LIGHT INTENSITY DATA │
│ AFTER SMOOTHING                  │
└──────────────────────────────────┘
                 │
 S130            ▼◄──────────────────────────┐
┌──────────────────────────────────┐         │
│ DETECTION OF PULSE EXISTING REGION│        │
│   DETECTION OF PULSE EXISTING     │        │
│   REGION FROM TIME DIFFERENTIAL   │        │
│   VALUE OF DATA                   │        │
└──────────────────────────────────┘         │
                 │                            │
 S140            ▼                            │
┌──────────────────────────────────┐         │
│ FITTING OF BELL-SHAPED FUNCTION   │        │
│   FITTING OF BELL-SHAPED FUNCTION │        │
│   TO TIME-SERIES LIGHT INTENSITY  │        │
│   DATA AFTER SMOOTHING IN PULSE   │        │
│   EXISTING REGION                 │        │
│   → CALCULATION OF COEFFICIENT    │        │
│   OF BELL-SHAPED FUNCTION         │        │
└──────────────────────────────────┘         │
                 │                            │
 S150            ▼                     ┌──────────────────────┐
┌──────────────────────────────────┐ │ TO NEXT PULSE        │
│ DETECTION OF PRESENCE OF ONE      │ │ DETECTION           │
│ PARTICLE                          │ └──────────────────────┘
│   THRESHOLD DETERMINATION WITH    │        ▲
│   REGARD TO COEFFICIENT OF        │        │
│   BELL-SHAPED FUNCTION            │        │
│   REMOVE NOISES                   │        │
└──────────────────────────────────┘        │
                 │                            │
 S160            ▼                    NO      │
         ◇─────────────────◇ ─────────────────┘
          ╲   DATA END?   ╱
           ◇─────────────◇
                 │ YES
 S170            ▼
┌──────────────────────────────────┐
│ ANALYSIS                         │
│   ABUNDANCE RATIO AND            │
│   CONCENTRATION                  │
│   CALCULATION AND THE LIKE       │
└──────────────────────────────────┘
```

33

# FIG. 4A

SCANNING DIRECTION

CV

PARTICLE TRACK

EX

OBSERVATION TARGET PARTICLE

# FIG. 4B

SCANNING DIRECTION

EX

CV

PARTICLE TRACK

α

β

# FIG. 4C

**DETECTION RESULTS (UNPROCESED)**

β          α

TIME→

**SMOOTHING**

SMOOTHING CURVE

TIME→

**TIME DIFFERENTIATION**          FIRST DIFFERENTIAL CURVE

START POINT          END          START POINT
OF PULSE          POINT          OF PULSE
OF PULSE

SMOOTHING          END POINT
CURVE          OF PULSE

TIME→

**FITTING OF BELL-SHAPED FUNCTION**

Wpeak    Ipeak          Wpeak    Ipeak

FITTING          FITTING
CURVE          CURVE

TIME→

(PHOTON COUNT / LIGHT INTENSITY)

# FIG. 5

LIGHT DETECTION AND
ANALYSIS PROCESS

S10
SETTING OF
END PARTICLE NUMBER (XE)

S20
SETTING OF
ANALYSIS TIME INTERVAL (t)

S25
STORAGE OF START TIME Ts

S30
LIGHT DETECTION AND DETECTION
OF NUMBER OF PARTICLES IN
ANALYSIS TIME INTERVAL (t) (FIG. 3)

S40
CALCULATION OF TOTAL NUMBER
OF DETECTED PARTICLES
$X(tn) = X(tn-1) + x$

S50
$XE \leq X(tn)$?

NO

YES

S60
STORAGE OF END TIME TE

S70
ANALYSIS, CONCENTRATION
CALCULATION AND THE LIKE

S58
UPDATE OF DISPLAY OF
Tr OR TE

S56
ESTIMATION OF
MEASUREMENT
REMAINING TIME (Tr) OR
ESTIMATION OF
MEASUREMENT
END TIME (TE)

S54
CALCULATION OF PARTICLE
DETECTION SPEED (v)

S52
UPDATE OF DISPLAY OF
TOTAL NUMBER X(tn) OF
DETECTED PARTICLES

# FIG. 6A

LIGHT DETECTION AND
ANALYSIS PROCESS

S10
SETTING OF
END PARTICLE NUMBER (XE)

S25
STORAGE OF START TIME Ts

S20'
COMPUTATION SETTING OF
ANALYSIS TIME INTERVAL (t)

S30
LIGHT DETECTION AND DETECTION
OF NUMBER OF PARTICLES IN
ANALYSIS TIME INTERVAL (t) (FIG. 3)

S40
CALCULATION OF TOTAL NUMBER
OF DETECTED PARTICLES
X(tn) = X(tn-1) + x

S50
XE ≤ X(tn)?

NO

YES

S60
STORAGE OF END TIME TE

S70
ANALYSIS, CONCENTRATION
CALCULATION AND THE LIKE

S58
UPDATE OF DISPLAY OF
Tr OR TE

S56
ESTIMATION OF
MEASUREMENT
REMAINING TIME (Tr) OR
ESTIMATION OF
MEASUREMENT
END TIME (TE)

S54
CALCULATION OF PARTICLE
DETECTION SPEED (v)

S52
UPDATE OF DISPLAY OF
TOTAL NUMBER X(tn) OF
DETECTED PARTICLES

# FIG. 6B

STEP 20'

S200　k = 0?　NO

　　　　YES

S210　t ← to

S220　X(tn) = 0?　NO

　　　　YES

S240　t ← Tr / (N-k)

S230　t ← m· t

S250　t < tmin?

　NO

　　　　YES

S260　t ← tmin

S270　k ← k + 1

RETURN

# FIG. 7

# FIG. 8

## INTERNATIONAL SEARCH REPORT

| International application No. |
| --- |
| PCT/JP2013/068406 |

A. CLASSIFICATION OF SUBJECT MATTER
*G01N21/64*(2006.01)i, *G01N21/76*(2006.01)i, *G01N33/558*(2006.01)i

According to International Patent Classification (IPC) or to both national classification and IPC

B. FIELDS SEARCHED

Minimum documentation searched (classification system followed by classification symbols)
G01N21/62-21/83, G01N33/48-33/98

Documentation searched other than minimum documentation to the extent that such documents are included in the fields searched

| | | | |
| --- | --- | --- | --- |
| Jitsuyo Shinan Koho | 1922–1996 | Jitsuyo Shinan Toroku Koho | 1996–2013 |
| Kokai Jitsuyo Shinan Koho | 1971–2013 | Toroku Jitsuyo Shinan Koho | 1994–2013 |

Electronic data base consulted during the international search (name of data base and, where practicable, search terms used)
JSTPlus/JMEDPlus/JST7580(JDreamIII)

C. DOCUMENTS CONSIDERED TO BE RELEVANT

| Category* | Citation of document, with indication, where appropriate, of the relevant passages | Relevant to claim No. |
| --- | --- | --- |
| A | WO 2012/014778 A1 (Olympus Corp.),<br>02 February 2012 (02.02.2012),<br>paragraphs [0010] to [0025], [0033] to [0044];<br>fig. 1 to 2<br>& US 2013/0122488 A1 & EP 2584343 A<br>& WO 2012/014778 A1 & CN 103026205 A | 1-14 |
| A | WO 2010/119098 A1 (WENNMALM, Stefan),<br>21 October 2010 (21.10.2010),<br>pages 3 to 7, 11 to 13, 22, 24; fig. 2, 9, 10<br>& US 2012/0050734 A1 & EP 2419715 A | 1-14 |
| P,A | WO 2013/031309 A1 (Olympus Corp.),<br>07 March 2013 (07.03.2013),<br>paragraphs [0011] to [0032], [0041] to [0048];<br>fig. 1 to 2<br>(Family: none) | 1-14 |

☐ Further documents are listed in the continuation of Box C. ☐ See patent family annex.

| | |
| --- | --- |
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>01 October, 2013 (01.10.13) | Date of mailing of the international search report<br>15 October, 2013 (15.10.13) |
| --- | --- |
| Name and mailing address of the ISA/<br>Japanese Patent Office | Authorized officer |
| Facsimile No. | Telephone No. |

Form PCT/ISA/210 (second sheet) (July 2009)

EP 2 913 660 A1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010119098 A
- WO 2011108369 A
- WO 2011108370 A
- WO 2011108371 A
- WO 2012014778 A

SEQUENCE LISTING

<110> OLYMPUS CORPORATION, Ltd.
<120> Method for detecting target particles
<130> 12P01842
<160> 4
<170> PatentIn version 3.1
<210> 1
<211> 43
<212> DNA
<213> Artificial Sequence
<220>
<223> Description of Artificial Sequence: Target DNA
<400> 1

gactgaatat aaacttgtgg agcctgggaa agtcccctca act 43

<210> 2
<211> 20
<212> DNA
<213> Artificial Sequence
<220>
<223> Description of Artificial Sequence: Probe DNA 1.
<400> 2

ccacaagttt atattcagtc 20

<210> 3
<211> 22
<212> DNA
<213> Artificial Sequence
<220>
<223> Description of Artificial Sequence: Probe DNA 2.
<400> 3

```
                    agttgagggg actttcccag gc 22
```

<210> 4
<211> 25
<212> DNA
<213> Artificial Sequence
<220>
<223> Description of Artificial Sequence: Probe DNA 3.
<400> 4

```
                    ccacaagttt atattcagtc ggggg

                    25
```

SEQUENCE LISTING

<110> OLYMPUS CORPORATION, Ltd.
<120> Method for detecting target particles
<130> 12P01842
<160> 4
<170> PatentIn version 3.1
<210> 1
<211> 43
<212> DNA
<213> Artificial Sequence
<220>
<223> Description of Artificial Sequence: Target DNA
<400> 1
gactgaatat aaacttgtgg agcctgggaa agtcccctca act          43
<210> 2
<211> 20
<212> DNA
<213> Artificial Sequence
<220>
<223> Description of Artificial Sequence: Probe DNA 1.
<400> 2
ccacaagttt atattcagtc          20
<210> 3
<211> 22
<212> DNA
<213> Artificial Sequence
<220>
<223> Description of Artificial Sequence: Probe DNA 2.
<400> 3
agttgagggg actttcccag gc          22

<210> 4
<211> 25
<212> DNA
<213> Artificial Sequence
<220>
<223> Description of Artificial Sequence: Probe DNA 3.
<400> 4
ccacaagttt atattcagtc ggggg          25